(19) Europäisches Patentamt
European Patent Office
Office européen des brevets

(11) Veröffentlichungsnummer: **0 310 954 B1**

# (12) EUROPÄISCHE PATENTSCHRIFT

(45) Veröffentlichungstag der Patentschrift: **22.11.90**

(51) Int. Cl.⁵: **C07C 69/734**, C07C 67/11, C07C 69/78, C07C 235/42, C07C 255/34, C07C 323/16, C07C 229/12, A01N 37/36, A01N 53/00, A01N 37/34, A01N 37/18

(21) Anmeldenummer: **88116173.1**

(22) Anmeldetag: **30.09.88**

(54) **Ortho-substituierte Carbonsäurebenzylester und Fungizide, die diese Verbindungen enthalten.**

(30) Priorität: **07.10.87 DE 3733870**

(43) Veröffentlichungstag der Anmeldung: **12.04.89 Patentblatt 89/15**

(45) Bekanntmachung des Hinweises auf die Patenterteilung: **22.11.90 Patentblatt 90/47**

(84) Benannte Vertragsstaaten: **AT BE CH DE ES FR GB GR IT LI NL SE**

(56) Entgegenhaltungen: **EP-A- 0 226 917**

(73) Patentinhaber: **BASF Aktiengesellschaft, Carl-Bosch-Strasse 38, D-6700 Ludwigshafen(DE)**

(72) Erfinder: **Schuetz, Franz, Dr., Budapester Strasse 45, D-6700 Ludwigshafen(DE)**
Erfinder: **Sauter, Hubert, Dr., Neckarpromenade 20, D-6800 Mannheim 1(DE)**
Erfinder: **Schirmer, Ulrich, Dr., Berghalde 79, D-6900 Heidelberg(DE)**
Erfinder: **Wolf, Bernd, Dr., Eichenstrasse 11, D-6704 Mutterstadt(DE)**
Erfinder: **Ammermann, Eberhard, Dr., Sachsenstrasse 3, D-6700 Ludwigshafen(DE)**
Erfinder: **Pommer, Ernst-Heinrich, Dr., Berliner Platz 7, D-6703 Limburgerhof(DE)**

EP 0 310 954 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue ortho-substituierte Carbonsäure-benzylester und Fungizide, welche diese Verbindungen enthalten.

Es ist bekannt, N-Tridecyl-2,6-dimethylmorpholin oder α-(2-Benzoyloxyphenyl)β-methoxyacrylsäure-methylester als Fungizide zu verwenden (DE 11 64 152, EP-178 826). Ihre fungiziden Wirkungen sind jedoch in manchen Fällen ungenügend.

Es wurde nun gefunden, daß neue ortho-substituierte Carbonsäure-benzylester der Formel

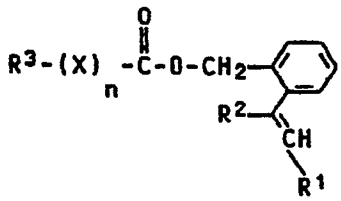

I

in der $R^1$ $C_1$-$C_4$-Alkoxy, $C_1$-$C_4$-Alkylthio, Halogen oder gegebenenfalls durch $C_1$-$C_4$-Alkyl einfach oder doppelt substituiertes Amino bedeutet,
$R^2$ $C_1$-$C_4$-Alkoxy-carbonyl, Cyano oder die Gruppe $CONH_2$ bedeutet,
$R^3$ Wasserstoff, Halogen, Cyano, Aryl, Aryloxy, wobei der aromatische Ring gegebenenfalls durch einen oder mehrere der folgenden Reste substituiert ist: $C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl, Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ einen gesättigten oder ungesättigten heterocyclischen Rest, $C_3$-$C_7$-Cycloalkyl, $C_5$-$C_6$-Cycloalkenyl, Adamantyl, Fluorenyl oder einen substituierten Cyclopropylrest bedeutet, der substituiert ist durch Methyl, Halogen (Chlor, Brom), $C_1$-$C_2$-Halogenalkyl (Trifluormethyl, Tetrabromethyl, Dichlor-dibromethyl), $C_3$-$C_4$-Alkenyl (Methylvinyl, Dimethylvinyl), $C_2$-$C_4$-Halogenalkenyl (Dichlorvinyl, Dichlorbutadienyl, Difluorvinyl, Trifluormethylvinyl), Methoxycarbonyl-$C_3C_4$-Alkenyl (Methyl-Methoxycarbonylvinyl), Cyclopentylidenmethyl, Phenyl, Halogenphenyl (Chlorphenyl), $C_1$-$C_2$-Alkoxyphenyl (Ethoxyphenyl), $C_1$-$C_4$-Alkylphenyl (tert. Butylphenyl),
X einen geradkettigen oder verzweigten, gegebenenfalls durch Halogen oder Hydroxy substituierten, gegebenenfalls auch ungesättigten $C_1$-$C_{12}$-Alkylenrest bedeutet und
n die Zahlen 0 oder 1 bedeutet,
eine ausgezeichnete fungizide Wirkung haben.

Die in der allgemeinen Formel aufgeführten Reste können beispielsweise folgende Bedeutung haben:

$R^1$ kann z.B. gegebenenfalls verzweigtes $C_1$-$C_4$-Alkoxy (z. B. Methoxy, Ethoxy, n- oder iso-Propoxy, n-, iso-, sec.- oder tert.-Butoxy), $C_1$-$C_4$-Alkylthio (z.B. Methylthio, Ethylthio, n- oder iso-Propylthio, n-, iso-, sec.- oder tert.-Butylthio), Halogen (z.B. Chlor, Brom), gegebenenfalls durch $C_1$-$C_4$-Alkyl, einfach oder doppelt substituiertes Amino (z.B. Amino, Methylamino, Methylethylamino, Dimethylamino, Diethylamino, Diisopropylamino) sein.

$R^2$ kann z.B. $C_1$-$C_4$-Alkoxycarbonyl (z. B. Methoxycarbonyl, Ethoxycarbonyl, n- oder iso-Propoxycarbonyl, n-, iso-, sec.- oder tert.-Butoxycarbonyl), Cyano oder die Gruppe $CONH_2$ sein.

$R^3$ kann z.B. Wasserstoff, Halogen (z. B. Fluor, Chlor, Brom), Cyano, Aryl (Phenyl, Naphthyl) oder Aryloxy (Phenyloxy) sein, wobei der aromatische Ring gegebenenfalls durch einen oder mehrere der folgenden Reste substituiert sein kann: $C_1$-$C_6$-Alkyl (z. B. Methyl, Ethyl, n- oder iso-Propyl, n-, iso-, sec.- oder tert.-Butyl, n-, iso-, sec.-, tert.-oder neo-Pentyl, Hexyl), $C_2$-$C_4$-Alkenyl (z.B. Vinyl, Allyl), $C_1$-$C_2$-Halogenalkyl (z.B. Difluormethyl, Trifluormethyl), $C_1$-$C_6$-Alkoxy (z. B. Methoxy, Ethoxy, iso-Propoxy, tert.-Butoxy), $C_1$-$C_4$-Alkoxy-$C_1$-$C_4$-alkyl (z. B. Methoxymethyl), Aryl (z. B. Phenyl), Aryl-$C_1$-$C_2$-alkyl (z.B. Benzyl), Aryloxy (z. B. Phenoxy), Aryloxy-$C_1$-$C_4$-alkyl (z. B. Phenoxymethyl, Phenoxyethyl), Aryloxy-$C_1$-$C_4$-alkoxy, Halogenaryloxy-$C_1$-$C_4$-alkoxy, (z.B. Phenoxymethoxy, Phenoxyethoxy, Phenoxypropoxy, 2-Chlor-phenoxy-ethoxy, 4-Chlor-phenoxy-ethoxy), Halogen (z. B. Fluor, Chlor, Brom, Jod), Halogen-$C_1$-$C_4$-alkoxy (z.B. 1,1,2,2-Tetrafluorethoxy), $C_1$-$C_4$-Alkylthio (z.B. Methylthio), Thiocyanato, Cyano, Nitro.

$R^3$ kann ferner bedeuten: einen gesättigten oder ungesättigten heterocyclischen Rest (z.B. Furyl, Pyrrolyl), $C_3$-$C_7$-Cycloalkyl, $C_5$-$C_6$-Cycloalkenyl, (z.B. Cyclopropyl, Cyclobutyl, Cyclopentyl, Cyclopentenyl, Cyclohexyl, Cyclohexenyl, Cycloheptyl), 1-Adamantyl, 9-Fluorenyl oder einen substituierten Cyclopropylrest, der substituiert ist durch Methyl, Halogen (Chlor, Brom), $C_1$-$C_2$-Halogenalkyl (Trifluormethyl, Tetrabromethyl, Dichlor-dibromethyl), $C_3$-$C_4$-Alkenyl (Methylvinyl, Dimethylvinyl), $C_2$-$C_4$-Halogenalkenyl (Dichlorvinyl, Dichlorbutadienyl, Difluorvinyl, Trifluormethylvinyl), Methoxycarbonyl-$C_3C_4$-Alkenyl (Methyl-Methoxycarbonylvinyl), Cyclopentylidenmethyl, Phenyl, Halogenphenyl (z.B. Fluorphenyl, Chlorphenyl, Bromphenyl, Dichlorphenyl), $C_1$-$C_2$-Alkoxyphenyl (z.B. Methoxyphenyl, Ethoxyphenyl), $C_1$-$C_4$-Alkylphenyl (z.B. Methylphenyl, Ethylphenyl, Butylphenyl, tert.-Butylphenyl), wie zum Beispiel:

2,2-Dimethyl-3-(2′,2′-dimethylvinyl)-cyclopropyl(A1)
2,2-Dimethyl-3-(2′,2′-dichlorvinyl)-cyclopropyl(A2)
2,2-Dimethyl-3-(2′,2′-dibromvinyl-cyclopropyl(A3)
2,2-Dimethyl-3-(2′-trifluormethyl-2′-chlorvinyl)-cyclopropyl(A4)
2,2-Dichlor-3,3-dimethyl-cyclopropyl(A5)
2,2,3,3-Tetramethyl-cyclopropyl(A6)
2,2-Dimethyl-3-(2′,2′-difluorvinyl)-cyclopropyl(A7)
2,2-Dimethyl-3-(2′-trifluormethyl-2′fluorvinyl)-cyclopropyl(A8)
2,2-Dimethyl-3-(2′-methyl-2′-methoxycarbonylvinyl)-cyclo-propyl(A9)
2,2-Dimethyl-3-(4′,4′-dichlorbutadienyl)-cyclopropyl(A10) 2,2-Dimethyl-3-(1′-brom-2′,2′,2′-tribromethyl)-cyclopropyl(A11) 2,2-Dimethyl-3-(1′-brom-2′,2′-dichlor-2′-bromethyl)-cyclopropyl(A12)
2,2-Dimethyl-3-Cyclopentylidenmethyl-cyclopropyl(A13)
1-(4′-Ethoxyphenyl)-2,2-dichlor-cyclopropyl(A14)
2,2-Dimethyl-3-(4′-tert.-Butylphenyl)-cyclopropyl (A15)

Der in der allgemeinen Formel I aufgeführte Rest X kann beispielsweise bedeuten: einen geradkettigen $C_1$-$C_{12}$-Alkylenrest (z. B. Methylen, Ethylen, Propylen, Butylen, Pentylen, Hexylen, Heptylen), einen verzweigten $C_1$-$C_{12}$-Alkylenrest (z. B. Methylmethylen, Dimethylmethylen, Ethylmethylen, n- oder iso-Propylmethylen, Methylethylen, Methylpropylen, Dimethylpropylen, Ethylpropylen, Methylbutylen, Dimethylbutylen, Ethylbutylen, n- oder iso-Propylbutylen, Methylpentylen, Dimethylpentylen, Trimethylpentylen, Methylhexylen, Dimethylhexylen, Trimethylhexylen, Ethylhexylen, n- oder iso-Propylhexylen, Methylheptylen), einen $C_2$-$C_8$-Alkenylenrest (z. B. Vinylen, Allylen, Methylallylen, Butenylen, Methylbutenylen), einen durch Halogen substituierten $C_1$-$C_{12}$-Alkylenrest (z. B. Chlormethylen, Dichlormethylen, Fluormethylen, Difluormethylen, Brommethylen, Dibrommethylen, Chlorethylen, Fluorethylen, Bromethylen, Fluorpropylen, Chlorpropylen, Brompropylen, Fluorbutylen, Chlorbutylen, Brombutylen), einen durch Halogen substituierten $C_3$-$C_4$-Alkenylenrest(z. B. Chlorvinylen, Dichlorvinylen), einen durch Hydroxy substituierten $C_1$-$C_8$-Alkylenrest (z.B. Hydroxymethylen, Hydroxyethylen).

$X_n$ bedeutet für den Fall n = 0 eine Einfachbindung.

Die neuen Verbindungen können z.B. hergestellt werden, indem man ein orthosubstituiertes Benzylbromid der allgemeinen Formel III, in der $R^1$ und $R^2$ die oben angegebene Bedeutung haben, mit einem Alkali-, Erdalkali- oder Ammoniumsalz einer Carbonsäure der Formel II, worin $R^3$, X und n die oben angegebene Bedeutung haben, in einem Lösungs- oder Verdünnungsmittel und gegebenenfalls unter Zusatz eines Katalysators zu den neuen Verbindungen umsetzt.

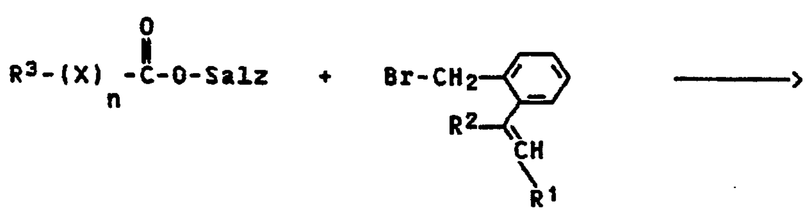

II III

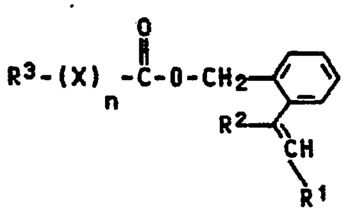

I

Die Herstellung von Carbonsäureestern aus Alkylhalogeniden und Carboxylaten ist an sich bekannt (vgl. z.B. Synthesis 1975, 805).

Als Lösungs- oder Verdünnungsmittel für die Reaktion von II mit III kommen Aceton, Acetonitril, Dimethylsulfoxid, Dioxan, Dimethylformamid, N-Methylpyrrolidon, N,N′-Dimethylpropylenharnstoff oder Pyridin in Betracht.

Außerdem kann es von Vorteil sein, der Reaktionsmischung einen Katalysator, wie z. B. Tetramethylethylendiamin, in einer Menge von 0,01 bis 10 % (Gew.-%), bezogen auf Verbindung III, zuzusetzen.

Die entsprechenden Umsetzungen können auch im Zweiphasensystem (z.B. Tetrachlorkohlenstoff/Wasser) durchgeführt werden. Als Phasentransferkatalysatoren kommen z.B. Trioctylpropylammoniumchlorid oder Cetyltrimethylammoniumchlorid in Betracht (vgl. Synthesis 1974, 867).

Die Carboxylate der allgemeinen Formel II erhält man in an sich bekannter Weise aus den entsprechenden Carbonsäuren durch Umsetzung mit Basen (z.B. Natriumhydroxid oder Kaliumhydroxid) in einem inerten Lösungsmittel (z.B. Ethanol).

Die verwendeten Carbonsäuren sind entweder bekannt oder können durch Verfahren analog zu bekannten Verfahren hergestellt werden. Entsprechende Herstellverfahren sind z.B. beschrieben in: Chem. Ber. 119 (1986) 3694; Synthesis 1987, 738; Angew. Chem. 93 (1981) 719.

Die Herstellung der ortho-substituierten Benzylbromide der allgemeinen Formel III kann beispielsweise durch Bromierung von ortho-substituierten Toluolen der allgemeinen Formel IV mit N-Bromsuccinimid erfolgen (Angew. Chem. 71 (1959) 349).

α-(2-Brommethylphenyl)-acrylester der allgemeinen Formel III ($R^1$ = Alkoxy, $R^2$ = Alkoxycarbonyl) sind bekannt aus DE-3 519 280, DE-3 545 318 und DE-3 545 319.

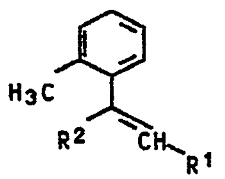

IV

Die Verbindungen der allgemeinen Formel IV können aufgrund ihrer C=C-Doppelbindung sowohl als E- als auch als Z-Isomere vorliegen. Die Isomeren können z.B. durch Chromatographie, fraktionierte Kristallisation oder Destillation in der üblichen Weise getrennt werden. Von der Erfindung werden sowohl die einzelnen isomeren Verbindungen als auch ihre Gemische umfaßt.

Die Verbindungen der allgemeinen Formel IVa ($R^1$ = Alkoxy, $R^2$ = Alkoxycarbonyl, Cyano) erhält man aus den Hydroxymethylenderivaten der allgemeinen Formel V, die im Gleichgewicht mit den Formylderivaten VI vorliegen können, mit einem Alkylierungsmittel (z.B. Dimethylsulfat) in Gegenwart einer Base (z.B. Kaliumcarbonat) in einem Verdünnungsmittel (z.B. Aceton). In den folgenden Formelbildern bedeutet "Alk" eine $C_1$-$C_4$-Alkylgruppe und X eine Abgangsgruppe (z. B. Methylsulfat).

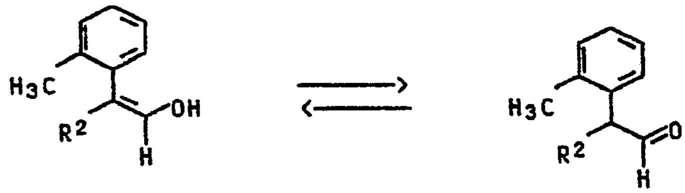

VI

V VI

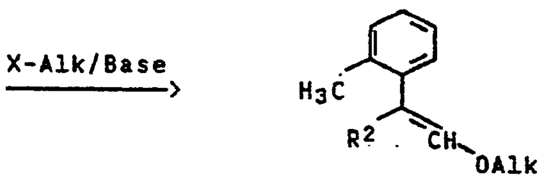


IVa
$R^2$ = Alkoxycarbonyl, Cyano

Zur Darstellung der Verbindungen der allgemeinen Formel IVb ($R^1$ = Alkylthio, $R^2$ = Alkoxycarbonyl, Cyano) werden die Hydroxymethylenderivate V, die auch im Gleichgewicht mit VI vorliegen können, zunächst mit Sulfonsäurechloriden, wie z.B. Methansulfochlorid (R'=Methyl), Trifluormethansulfochlorid (R' = Trifluormethyl) oder p-Toluolsulfonsäurechlorid (R' = p-Methylphenyl), in Gegenwart von Basen (z.B. Triethylamin) zu Verbindungen der allgemeinen Formel VII umgesetzt. Anschließend erhält man die gewünschten Verbindungen IV b durch Umsetzung von VII mit Alkylthiolaten $AlkS^{\ominus}$, wie z.B. Natriumthiomethylat.

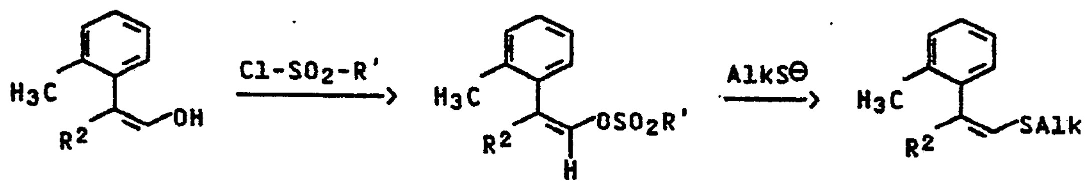


V VII IVb

$R^2$ = Alkoxycarbonyl, Cyano

Verbindungen der allgemeinen Formel IVc ($R^1$ = Halogen, $R^2$ = Alkoxycarbonyl, Cyano) werden dadurch gewonnen, daß man die Hydroxymethylenverbindungen V, die im Gleichgewicht mit den Formylderivaten VI vorliegen können, mit anorganischen Säurechloriden (z.B. Phosphorpentachlorid) umsetzt (vgl. z.B. Chem. Ber, 51 (1918) 1366).

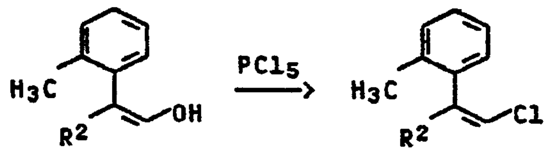


V IVc

$R^2$ = Alkoxycarbonyl, Cyano

Die Darstellung der Verbindungen der allgemeinen Formel IV d ($R^1$ = Alkylamino bzw. Dialkylamino, $R^2$ = Alkoxycarbonyl, Cyano) erfolgt durch Umsetzung der Hydroxymethylenderivate V, die auch im Gleichgewicht mit VI vorliegen können, mit primären oder sekundären Aminen. Alternativ können auch die Alkalisalze von V mit den Hydrochloriden von primären oder sekundären Aminen unter Freisetzung von Natriumchlorid umgesetzt werden (vgl. dazu Ann. Chim. [10] 18 (1932) 103).

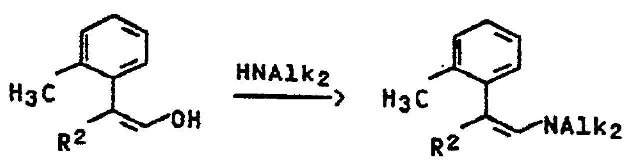


V IVd

$R^2$ = Alkoxycarbonyl, Cyano

Zu Verbindungen der Formel IV d kommt man auch, wenn man 2-Methylphenyl-essigsäurealkylester VIII bzw. 2-Methylphenyl-acetonitril IX mit Dialkylformamiddialkylacetalen oder mit Aminal-alkylestern umsetzt (vgl. z.B. Chem. Ber. 97 (1964) 3396).

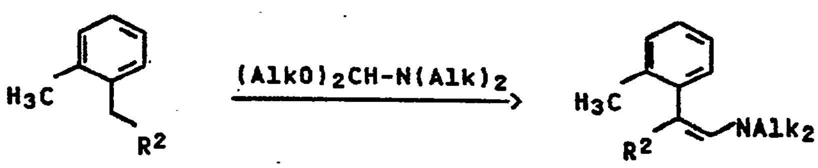


IVd

VIII: $R^2$ = Alkoxycarbonyl
IX: $R^2$ = Cyano

Die neuen Verbindungen der Formel I mit $R^2$ = $CONH_2$ erhält man ausgehend von den entsprechenden Derivaten mit $R^2$ = Cyano durch alkalische Verseifung (vgl. dazu Synthesis 1980, 243).

Die als Ausgangsverbindungen benötigten Hydroxymethylenderivate der allgemeinen Formel V, in der $R^2$ Alkoxycarbonyl oder Cyano bedeutet, erhält man aus 2-Methylphenyl-essigsäurealkylester VIII

bzw. aus 2-Methylphenylacetonitril IX durch Umsetzung mit Ameisensäuremethylester unter Verwendung einer Base (z. B. Natriumhydrid) in einem inerten Lösungsmittel, wie z.B Diethylether oder Tetrahydrofuran (vgl. dazu Ann. Chem. 424 (1921) 214).

Das folgende Beispiel soll die Herstellung der neuen Wirkstoffe erläutern.

Beispiel 1

α-(2-Benzoyloxymethylphenyl)-β-methoxyacrylsäuremethylester

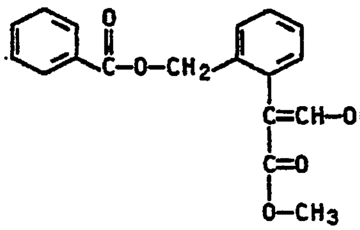

12,2 g (0,1 mol) Benzoesäure und 5,6 g (0,1 mol) Kaliumhydroxid werden in 150 ml Ethanol gelöst und zwei Stunden bei Raumtemperatur (20°C) gerührt. Der ausgefallene, weiße Niederschlag wird abgesaugt, mit Diethylether gewaschen und in 300 ml Dimethylformamid suspendiert. Anschließend werden 28,5 g (0,1 mol) alpha-(2-Brommethylphenyl)-β-methoxyacrylsäuremethylester zugegeben. Man rührt 24 Stunden bei Raumtemperatur, engt anschließend das Reaktionsgemisch ein und nimmt den Rückstand in Methylenchlorid auf. Die organische Phase wird mit Wasser gewaschen, über $MgSO_4$ getrocknet und eingeengt. Das erhaltene Öl wird an Kieselgel (Cyclohexan:Essigester 10:1) chromatographiert. Man erhält 25,4 g (78%) der Titelverbindung als farbloses, zähes Öl. (Verbindung Nr. 83)

Beispiel 2

α-[2-(1′-ortho-Chlorphenyl)-cyclopropylcarbonyloxymethylphenyl]-β-methoxy-acrylsäuremethylester

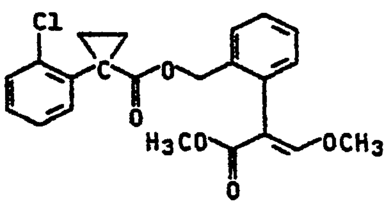

a) Eine Mischung aus 30,0 g (200 mmol) 2-Chlorbenzylcyanid und 80,0 g (426 mmol) Dibromethan werden langsam zu einer Lösung von 40,0 g Triethylbutylammoniumchlorid in 200 ml Natronlauge (30 %ig) getropft. Man rührt zwei Stunden bei 80°C, läßt abkühlen, hydrolysiert mit 500 ml Eiswasser und extrahiert mit Diethylether. Die organische Phase wird mit Ammoniumchloridlösung und Wasser gewaschen, über $MgSO_4$ getrocknet und eingeengt. Das erhaltene Öl wird durch Destillation (97°C, 0,4 mbar) gereinigt. Man erhält 20,0 g (56%) 1-(2′-Chlorphenyl)-cyclopropylnitril als farblose Flüssigkeit.

b) 11,0 g (62 mmol) 1-(2′-Chlorphenyl)-cyclopropylnitril und 10,0 g (180 mmol) Kaliumhydroxid werden in 130 ml Diethylenglykol zwei Stunden unter Rückfluß erhitzt. Man läßt abkühlen, hydrolysiert mit 200 ml Wasser und extrahiert mit Diethylether. Die wäßrige Phase wird mit HCl (verd.) angesäuert und mit Methylenchlorid extrahiert. Die vereinigten Methylenchlorid-Phasen werden über $MgSO_4$ getrocknet, eingeengt und mit Hexan überschichtet. Durch Anreiben erhält man 9,9 g (81%) 1-(2′-Chlorphenyl)-cyclopropancarbonsäure in Form farbloser Kristalle (Fp.: 160°C).

c) 9,8 g (50 mmol) 1-(2′-Chlorphenyl)-cyclopropancarbonsäure und 2,8 g (50 mmol) Kaliumhydroxid werden in 100 ml Ethanol gelöst und eine Stunde bei Raumtemperatur (20°C) gerührt. Der ausgefallene, weiße Niederschlag wird abgesaugt, mit Diethylether gewaschen und in 300 ml N-Methylpyrrolidon suspendiert. Anschließend werden 14,3 g (50 mmol) α-(2-Brommethylphenyl)-β-methoxyacrylsäure-methylester zugegeben. Man rührt zwei Stunden bei 70°C, läßt abkühlen, hydrolysiert mit 150 ml Wasser und extrahiert mit Methyl-tert.-butylether. Die organische Phase wird mit Wasser gewaschen, über $MgSO_4$ getrocknet und eingeengt. Das erhaltene Öl wird mit Hexan überschichtet und durch Anreiben kristallisiert. Man erhält 12,8 g (64%) der Titelverbindung in Form weißer Kristalle (Fp.: 100 - 101°C).

In entsprechender Weise lassen sich folgende Verbindungen herstellen:

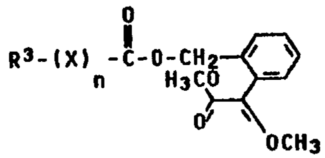

I

Tabelle 1: Verbindungen der Formel I
($R^1$=$OCH_3$, $R_2$=$CO_2CH_3$)
Die Konfigurationsangabe bezieht sich auf die β-Methoxy-acrylestergruppe

| Nr. | $R^3$ | $(X)_n$ | Konfiguration | $^1$H-NMR-Daten ($CDd_3$), δ in [ppm] | Fp. (°C) |
|---|---|---|---|---|---|
| 1 | H | $-CH_2-$ | E | 2,05(s,3H); 3,69(s,3H); 3,80(s,3H); 5,02(s,2H) 7,35(m,4H); 7,57(s,1H). | Oel |
| 2 | H | $-CH_2-CH_2-$ | E | | |
| 3 | H | $-CH_2-CH(CH_3)-$ | E | | |
| 4 | H | $-CH_2-C(CH_3)_2-$ | E | | 74 - 75 |
| 5 | H | $-CH=CH-$ | E | | |
| 6 | H | $-CH=C(CH_3)-$ | E | | 61 - 62 |
| 7 | H | $-C \equiv C-$ | E | | Oel |
| 8 | H | $-CH_2-CH_2-CH_2-$ | E | | |
| 9 | H | $-CH_2-CH_2-CH(CH_3)-$ | E | | |
| 10 | H | $-CH_2-CH(CH_3)-CH_2-$ | E | | |
| 11 | H | $-CH_2-CH_2-C(CH_3)_2-$ | E | | Oel |
| 12 | H | $-CH_2-C(CH_3)_2-CH_2-$ | E | | |
| 13 | H | $-CH_2-CH_2-C(C_2H_5)_2-$ | E | | |
| 14 | H | $-CH=CH-CH_2-$ | E | | |
| 15 | H | $-CH_2-CH=CH-$ | E | | |
| 16 | H | $-CH_2-C(CH_3)=CH-$ | E | | |
| 17 | H | $-CH_2-CH=C(CH_3)-$ | E | | |
| 18 | H | $-(CH_2)_4-$ | E | | |
| 19 | H | $-CH_2-CH_2-CH_2-CH(CH_3)-$ | E | | |

| Nr. | $R^3$ | $(X)_n$ | Konfiguration | $^1H$-NMR | Fp. (°C) |
|---|---|---|---|---|---|
| 20 | H | $-CH_2-CH(CH_3)-CH_2-CH_2-$ | E | | |
| 21 | H | $-(CH_2)_3-C(CH_3)_2-$ | E | | |
| 22 | H | $-(CH_2)_3-CH(C_2H_5)-$ | E | | |
| 23 | H | $-(CH_2)_3-CH(n-C_3H_7)-$ | E | | Oel |
| 24 | H | $-CH_2-CH=CH-CH_2-$ | E | | |
| 25 | H | $-CH_2-C(CH_3)=CH-CH_2-$ | E | | |
| 26 | H | $-(CH_2)_5-$ | E | | |
| 27 | H | $-(CH_2)_4-CH(CH_3)-$ | E | | |
| 28 | H | $-(CH_2)_4-CH(C_2H_5)-$ | E | | Oel |
| 29 | H | $-(CH_2)_3-CH(CH_3)-CH_2-$ | E | | |
| 30 | H | $-CH_2-CH=CH-CH=CH-$ | E | | |
| 31 | H | $-CH_2-C(CH_3)=CH-CH=CH-$ | E | | |
| 32 | H | $-(CH_2)_6-$ | E | | Oel |
| 33 | H | $-(CH_2)_5-CH(CH_3)-$ | E | | |
| 34 | H | $-(CH_2)_4-CH(CH_3)-CH_2-$ | E | 0,88(t,3H); 0,93(d,3H); 1,29(m,4H); 1,96(m,1H); 2,13(m,1H); 2,33(m,1H); 3,69(s,3H); 3,79(s,3H); 5,03(s,2H); 7,32(m,4H); 7,57(s,1H). | Oel |
| 35 | H | $-(CH_2)_5-CH(n-C_3H_7)-$ | E | | Oel |
| 36 | H | $-(CH_2)_7-$ | | | |
| 37 | H | $-(CH_2)_6-CH(CH_3)-$ | E | | |
| 38 | H | $-(CH_2)_5-CH(CH_3)-CH_2-$ | E | | |
| 39 | H | $-(CH_2)_6-C(CH_3)_2-$ | E | | |
| 40 | H | $-(CH_2)_8-$ | E | | |
| 41 | H | $-(CH_2)_9-$ | E | | |
| 42 | H | $-(CH_2)_{10}-$ | E | | |
| 43 | H | $-CHCl-$ | E | | |

| Nr. | $R^3$ | $(X)_n$ | Konfiguration | $^1H$-NMR | Fp. (°C) |
|---|---|---|---|---|---|
| 44 | H | $-CCl_2-$ | E | | |
| 45 | Cl | $-CCl_2-$ | E | | |
| 46 | H | $-CHBr-$ | E | | |
| 47 | H | $-CBr_2-$ | E | | |
| 48 | Br | $-CBr_2-$ | E | | |
| 49 | H | $-CHF-$ | E | | |
| 50 | H | $-CF_2-$ | E | | |
| 51 | F | $-CF_2-$ | E | | |
| 52 | H | $-CH=CCl-$ | E | | |
| 53 | H | $-CCl=CCl-$ | E | | |
| 54 | Cl | $-C(CH_3)_2-$ | E | | |
| 55 | Br | $-C(CH_3)_2-$ | E | | |
| 56 | H | $-CHCl-CH(CH_3)-$ | E | | |
| 57 | H | $-CHCl-C(CH_3)_2-$ | E | | |
| 58 | H | $-CHBr-CH(CH_3)-$ | E | | |
| 59 | Br | $-C(C_2H_5)_2-$ | E | | |
| 60 | H | $-CH(OH)-$ | E | | |
| 61 | H | $-CH_2-CH(OH)-$ | E | | |
| 62 | H | $-CH_2-CH_2-CH(OH)-$ | E | | |
| 63 | H | $-CH_2-CH(OH)-CH_2-$ | E | | |
| 64 | H | $-CH(OH)-CH_2-$ | E | | |
| 65 | H | $-CH(OH)-C(CH_3)_2-$ | E | | |
| 66 | H | $-CH_2-C(OH)(CH_3)-$ | E | | |
| 67 | H | $-CH_2-CH(CH_3)-CH(OH)-$ | E | | |
| 68 | H | $-CH=CH-CH(OH)-$ | E | | |
| 69 | H | $-CH=CH-CH_2-CH(OH)-$ | E | | |
| 70 | CN | $-CH_2-$ | E | | |
| 71 | Cyclopropyl | - | E | | Oel |
| 72 | Cyclobutyl | - | E | | |

| Nr. | $R^3$ | $(X)_n$ | Konfiguration | $^1H$-NMR | Fp. (°C) |
|---|---|---|---|---|---|
| 73 | Cyclopentyl | - | E | | Oel |
| 74 | Cyclohexyl | - | E | | |
| 75 | Adamantyl | - | E | | |
| 76 | 9-Fluorenyl | - | E | | |
| 77 | Cyclopentyl | $-CH_2-$ | E | | |
| 78 | 3-Cyclopentenyl | $-CH_2-$ | E | | |
| 79 | Cyclohexyl | $-CH_2-$ | E | | Oel |
| 80 | Cyclopentyl | $-CH_2-CH_2-$ | E | | |
| 81 | Cyclohexyl | $-CH_2-CH_2-$ | E | | Oel |
| 82 | Cyclohexyl | $-(CH_2)_3-$ | E | | Oel |
| 83 | $C_6H_5$ (=Phenyl) | - | E | 3,60(s,3H); 3,76(s,3H); 5,27(s,3H); 7,50(m,9H); 7,57(s,1H). | Oel |
| 84 | $2-CH_3-C_6H_4$ | - | E | | |
| 85 | $3-CH_3-C_6H_4$ | - | E | | |
| 86 | $4-CH_3-C_6H_4$ | - | E | | |
| 87 | $2,3-(CH_3)_2-C_6H_3$ | - | E | | |
| 88 | $2,4-(CH_3)_2-C_6H_3$ | - | E | | |
| 89 | $2,6-(CH_3)_2-C_6H_3$ | - | E | | |
| 90 | $3,4-(CH_3)_2-C_6H_3$ | - | E | | |
| 91 | $3,5-(CH_3)_2-C_6H_3$ | - | E | | |
| 92 | $2,4,6-(CH_3)_3-C_6H_2$ | - | E | | |
| 93 | $4-t-C_4H_9-C_6H_4$ | - | E | | |
| 94 | $2-C_6H_5-C_6H_4$ | - | E | | |
| 95 | $4-C_6H_5-C_6H_4$ | - | E | | |
| 96 | $2-Benzyl-C_6H_4$ | - | E | | |
| 97 | $4-Benzyl-C_6H_4$ | - | E | | |

| Nr. | $R^3$ | $(X)_n$ | Konfiguration | $^1H$-NMR | Fp. (°C) |
|---|---|---|---|---|---|
| 98 | 2-Cl-$C_6H_4$ | - | E | 3,65(s,3H); 3,83(s,3H); 5,30(s,2H); 7,55(m,8H); 7,63(s,1H). | Oel |
| 99 | 3-Cl-$C_6H_4$ | - | E | | |
| 100 | 4-Cl-$C_6H_4$ | - | E | | |
| 101 | 2,4-$Cl_2$-$C_6H_3$ | - | E | 3,62(s,3H); 3,73(s,3H); 5,27(s,2H); 7,48(m,7H); 7,68(s,1H). | Oel |
| 102 | 2,5-$Cl_2$-$C_6H_3$ | - | E | | |
| 103 | 2,6-$Cl_2$-$C_6H_3$ | - | E | | |
| 104 | 3,4-$Cl_2$-$C_6H_3$ | - | E | | |
| 105 | 3,5-$Cl_2$-$C_6H_3$ | - | E | | |
| 106 | 2,4,5-$Cl_3$-$C_6H_2$ | - | E | | |
| 107 | 2,3,4,5,6-$Cl_5$-$C_6$ | - | E | | |
| 108 | 2-F-4-Cl-$C_6H_3$ | - | E | | |
| 109 | 2-F-$C_6H_4$ | - | E | 3,63(s,3H); 3,73(s,3H); 5,29(s,2H); 7,49(m,8H); 7,60(s,1H); | 68 - 69 |
| 110 | 3-F-$C_6H_4$ | - | E | | 68 - 70 |
| 111 | 4-F-$C_6H_4$ | - | E | | Oel |
| 112 | 2,4-$F_2$-$C_6H_3$ | - | E | | 63 - 65 |
| 113 | 2,6-$F_2$-$C_6H_3$ | - | E | | |
| 114 | 2,3,4,5,6-$F_5$-$C_6$ | - | E | | |
| 115 | 2-$CF_3$-$C_6H_4$ | - | E | | |
| 116 | 3-$CF_3$-$C_6H_4$ | - | E | | |
| 117 | 4-$CF_3$-$C_6H_4$ | - | E | | Oel |
| 118 | 2-$OCH_3$-$C_6H_4$ | - | E | | |
| 119 | 3-$OCH_3$-$C_6H_4$ | - | E | | |
| 120 | 4-$OCH_3$-$C_6H_4$ | - | E | | |
| 121 | 2-Phenoxy-$C_6H_4$ | - | E | | |
| 122 | 3-Phenoxy-$C_6H_4$ | - | E | | |
| 123 | 4-Phenoxy-$C_6H_4$ | - | E | | |

| Nr. | $R^3$ | $(X)_n$ | Konfiguration | $^1H$-NMR | Fp. (°C) |
|---|---|---|---|---|---|
| 124 | 4-Ethoxy-$C_6H_4$ | - | E | | |
| 125 | 2-Phenoxyethoxy-$C_6H_4$ | - | E | | |
| 126 | 2-(2'-Cl-Phenoxyethoxy)-$C_6H_4$ | - | E | | |
| 127 | 2-(3'-Cl-Phenoxyethoxy)-$C_6H_4$ | - | E | | |
| 128 | 2-(4'-Cl-Phenoxyethoxy)-$C_6H_4$ | - | E | | |
| 129 | 3-Phenoxyethoxy-$C_6H_4$ | - | E | | |
| 130 | 3-(4'-Cl-Phenoxyethoxy)-$C_6H_4$ | - | E | | |
| 131 | 4-Phenoxyethoxy-$C_6H_4$ | - | E | | |
| 132 | 2-Phenoxypropxy-$C_6H_4$ | - | E | | |
| 133 | 3-Phenoxypropoxy-$C_6H_4$ | - | E | | |
| 134 | 4-Phenoxypropoxy-$C_6H_4$ | - | E | | |
| 135 | $C_6H_5$ | $-CH_2-$ | E | 3,59(s,2H); 3,63(s,3H); 3,65(s,3H); 7,28(m,9H); 7,52(s,1H). | Oel |
| 136 | 2-$CH_3$-$C_6H_4$ | $-CH_2-$ | E | | |
| 137 | $C_6H_5$ | $-CHCH_3-$ | E | | |
| 138 | 4-Phenyl-$C_6H_4$ | $-CH_2-$ | E | | |
| 139 | 2-F-$C_6H_4$ | $-CH_2-$ | E | | |
| 140 | 3-F-$C_6H_4$ | $-CH_2-$ | E | | |
| 141 | 4-F-$C_6H_4$ | $-CH_2-$ | E | | |
| 142 | 2-Cl-$C_6H_4$ | $-CH_2-$ | E | | |
| 143 | 3-Cl-$C_6H_4$ | $-CH_2-$ | E | | |
| 144 | 4-Cl-$C_6H_4$ | $-CH_2-$ | E | | |
| 145 | 2,4-$Cl_2$-$C_6H_3$ | $-CH_2-$ | E | | |
| 146 | 2,6-$Cl_2$-$C_6H_3$ | $-CH_2-$ | E | | |
| 147 | 2-Cl-4-F-$C_6H_3$ | $-CH_2-$ | E | | |
| 148 | 2-Ethoxy-$C_6H_4$ | $-CH_2-$ | E | | |
| 149 | 4-Ethoxy-$C_6H_4$ | $-CH_2-$ | E | | |
| 150 | 2-$OCH_3$-$C_6H_4$ | $-CH_2-$ | E | | |
| 151 | 4-$OCH_3$-$C_6H_4$ | $-CH_2-$ | E | | |

| Nr. | $R^3$ | $(X)_n$ | Konfiguration | $^1$H-NMR | Fp. (°C) |
|---|---|---|---|---|---|
| 152 | $4-t-C_4H_9-C_6H_4$ | $-CH_2-$ | E | | |
| 153 | $C_6H_5$ | $-CH(iso-C_3H_7)-$ | E | | |
| 154 | $4-Cl-C_6H_4$ | $-CH(iso-C_3H_7)-$ | E | 0,75(d,3H); 1,07(d,3H); 2,33(m,1H); 3,22(d,1H); 2,73(s,3H); 3,83(s,3H); 5,03(dd,2H); 7,35(m,8H); 7,58(s,1H). | Oel |
| 155 | $4-F-C_6H_4$ | $-CH(iso-C_3H_7)-$ | E | | |
| 156 | $4-OCF_2H-C_6H_4$ | $-CH(iso-C_3H_7)-$ | E | | |
| 157 | $C_6H_5$ | $-CH(OH)-$ | E | | Oel |
| 158 | $2-OCH_3-C_6H_4$ | $-CH(OH)-$ | E | | |
| 159 | $3-OCH_3-C_6H_4$ | $-CH(OH)-$ | E | | |
| 160 | $4-OCH_3-C_6H_4$ | $-CH(OH)-$ | E | | |
| 161 | $4-Cl-C_6H_4$ | $-CH(OH)-$ | E | | Oel |
| 162 | $C_6H_5$ | $-CH(CH_2OH)-$ | E | | |
| 163 | $C_6H_5$ | $-CH_2-CH_2-$ | E | | |
| 164 | $C_6H_5$ | $-CH(CH_3)-CH_2-$ | E | | |
| 165 | $C_6H_5$ | $-CH_2-CH(CH_3)-$ | E | | |
| 166 | $C_6H_5$ | $-CH(CH_3)-CH(CH_3)-$ | E | | |
| 167 | $C_6H_5$ | $-CH(C_6H_5)-CH_2-$ | E | 3,12(d,2H); 3,72(s,3H); 3,83(s,3H); 4,62(t,1H); 4,99(s,2H); 7,31(m,14H); 7,60(s,1H). | Oel |
| 168 | $4-t-C_4H_9-C_6H_4$ | $-CH_2-CH_2-$ | E | | |
| 169 | $4-t-C_4H_9-C_6H_4$ | $-CH_2-CH(CH_3)-$ | E | 1,16(d,3H); 1,30(s,9H); 2,72(m,2H); 3,02(m,1H); 3,70(s,3H); 3,82(s,3H); 5,00(s,2H); 7,18(m,8H); 7,60)s,1H). | Oel |
| 170 | $2-Cl-C_6H_4$ | $-CH_2-CH_2-$ | E | | |
| 171 | $3-Cl-C_6H_4$ | $-CH_2-CH_2-$ | E | | |
| 172 | $4-Cl-C_6H_4$ | $-CH_2-CH_2-$ | E | | |

| Nr. | $R^3$ | $(X)_n$ | Konfiguration | $^1H$-NMR | Fp. ($^oC$) |
|---|---|---|---|---|---|
| 173 | $2-F-C_6H_4$ | $-CH_2-CH_2-$ | E | | |
| 174 | $3-F-C_6H_4$ | $-CH_2-CH_2-$ | E | | |
| 175 | $4-F-C_6H_4$ | $-CH_2-CH_2-$ | E | | |
| 176 | $2-OCH_3-C_6H_4$ | $-CH_2-CH_2-$ | E | | |
| 177 | $4-OCH_3-C_6H_4$ | $-CH_2-CH_2-$ | E | | |
| 178 | $C_6H_5$ | -CH=CH- | E | | |
| 179 | $2-Cl-C_6H_4$ | -CH=CH- | E | 3,67(s,3H); 3,76(s,3H); 5,19(s,2H); 6,44(d,1H); 7,33(m,8H); 7,60(s,1H); 8,09(d,1H). | Oel |
| 180 | $3-Cl-C_6H_4$ | -CH=CH- | E | | |
| 181 | $4-Cl-C_6H_4$ | -CH=CH- | E | | |
| 182 | $2,6-Cl_2-C_6H_3$ | -CH=CH- | E | | |
| 183 | $2,4-Cl_2C_6H_3$ | -CH=CH- | E | | |
| 184 | $2-F-C_6H_4$ | -CH=CH- | E | | Oel |
| 185 | $3-F-C_6H_4$ | -CH=CH- | E | | |
| 186 | $4-F-C_6H_4$ | -CH=CH- | E | | |
| 187 | $2-CF_3-C_6H_4$ | -CH=CH- | E | | |
| 188 | $4-CF_3-C_6H_4$ | -CH=CH- | E | | |
| 189 | $2-CH_3-C_6H_4$ | -CH=CH- | E | | |
| 190 | $4-CH_3-C_6H_4$ | -CH=CH- | E | | |
| 191 | $4-i-C_3H_7-C_6H_4$ | -CH=CH- | E | | Oel |
| 192 | $4-t-C_4H_9-C_6H_4$ | -CH=CH- | E | | Oel |
| 193 | $2-OCH_3-C_6H_4$ | -CH=CH- | E | | |
| 194 | $3-OCH_3-C_6H_4$ | -CH=CH- | E | | |
| 195 | $4-OCH_3-C_6H_4$ | -CH=CH- | E | | |
| 196 | 2-Phenoxy-$C_6H_4$ | -CH=CH- | E | | |
| 197 | 3-Phenoxy-$C_6H_4$ | -CH=CH- | E | | |
| 198 | 4-Phenoxy-$C_6H_4$ | -CH=CH- | E | | |
| 199 | $C_6H_5$ | $-(CH_2)_3-$ | E | | |

| Nr. | $R^3$ | $(X)_n$ | Konfiguration | $^1H$-NMR | Fp. (°C) |
|---|---|---|---|---|---|
| 200 | $C_6H_5$ | $-CH(CH_3)-CH_2-CH_2-$ | E | | |
| 201 | $C_6H_5$ | $-CH_2-CH(CH_3)-CH_2-$ | E | | |
| 202 | $C_6H_5$ | $-CH_2-CH_2-CH(CH_3)-$ | E | | |
| 203 | $2-Cl-C_6H_4$ | $-(CH_2)_3-$ | E | | |
| 204 | $4-Cl-C_6H_4$ | $-(CH_2)_3-$ | E | | |
| 205 | $2-OCH_3-C_6H_4$ | $-(CH_2)_3-$ | E | | |
| 206 | $4-OCH_3-C_6H_4$ | $-(CH_2)_3-$ | E | | |
| 207 | $4-t-C_4H_9-C_6H_4$ | $-(CH_2)_3-$ | E | | |
| 208 | $C_6H_5$ | $-CH=CH-CH_2-$ | E | 3,28(d,2H); 3,73(s,3H); 3,83(s,3H); 5,17(s,2H); 6,33(m,1H); 6,52(d,1H); 7,33(m,9H); 7,62 (s,1H). | Oel |
| 209 | $C_6H_5$ | $-(CH_2)_4-$ | E | 1,65(m,4H); 2,35(t,2H); 2,60(t,2H); 3,65(s,3H); 3,70(s,3H); 5,03(s,2H); 7,29(m,9H); 7,53(s,1H). | Oel |
| 210 | $2-Cl-C_6H_4$ | $-(CH_2)_4-$ | E | | |
| 211 | $4-Cl-C_6H_4$ | $-(CH_2)_4-$ | E | | Oel |
| 212 | $2-OCH_3-C_6H_4$ | $-(CH_2)_4-$ | E | | |
| 213 | $4-OCH_3-C_6H_4$ | $-(CH_2)_4-$ | E | | |
| 214 | $4-CF_3-C_6H_4$ | $-(CH_2)_4-$ | E | | |
| 215 | $2-CH_3-C_6H_4$ | $-(CH_2)_4-$ | E | | |
| 216 | $4-CH_3-C_6H_4$ | $-(CH_2)_4-$ | E | | |
| 217 | $C_6H_5$ | $-CH_2-CH_2-CH(CH_3)-CH_2-$ | E | | |
| 218 | $C_6H_5$ | $-(CH_2)_5-$ | E | | Oel |
| 219 | $2-CH_3-C_6H_4$ | $-(CH_2)_5-$ | E | | |
| 220 | $4-CH_3-C_6H_4$ | $-(CH_2)_5-$ | E | | |
| 221 | $2-Cl-C_6H_4$ | $-(CH_2)_5-$ | E | | |
| 222 | $4-Cl-C_6H_4$ | $-(CH_2)_5-$ | E | | |

| Nr. | $R^3$ | $(X)_n$ | Konfiguration | $^1$H-NMR | Fp. (°C) |
|---|---|---|---|---|---|
| 223 | 2-$OCH_3$-$C_6H_4$ | -$(CH_2)_5$- | E | | |
| 224 | 4-$OCH_3$-$C_6H_4$ | -$(CH_2)_5$- | E | | |
| 225 | 4-$CF_3$-$C_6H_4$ | -$(CH_2)_5$- | E | | |
| 226 | $C_6H_5$ | -$CH_2$-$CH_2$-$CH_2$-CH($CH_3$)-$CH_2$- | E | 0,95(d,3H); 1,25(m,1H); 1,37(m,1H); 1,63(m,2H); 2,00(m,1H); 2,13(m,1H); 2,32(m,1H); 2,57(m,2H); 3,67(s,3H); 3,75(s,3H); 5,03(s,2H); 7,29(m,9H); 7,56(s,H). | Oel |
| 227 | $C_6H_5$ | -$CH_2$-CH($CH_3$)-$CH_2$-CH($CH_3$)-$CH_2$- | E | | |
| 228 | 4-t-$C_4H_9$-$C_6H_4$ | -$CH_2$-CH($CH_3$)-$CH_2$-CH($CH_3$)-$CH_2$)- | E | 0,83, 0,88, 0,96(3d,6H); 1,19(m,2H); 1,31(s,9H); 1,79(m,1H); 2,20(m,4H); 2,60(m,1H); 3,69(s,3H); 3,81(s,3H); 5,03(s,2H); 7,25(m,8H); 7,57(s,1H). | Oel |
| 229 | $C_6H_5$ | -$(CH_2)_6$- | E | | |
| 230 | $C_6H_5$ | -$(CH_2)_4$-CH($CH_3$)-$CH_2$- | E | | |
| 231 | $C_6H_5$-O- | -$CH_2$- | E | | |
| 232 | 2-Cl-$C_6H_4$-O- | -$CH_2$- | E | | |
| 233 | 3-Cl-$C_6H_4$-O- | -$CH_2$- | E | | |
| 234 | 4-Cl-$C_6H_4$-O- | -$CH_2$- | E | | |
| 235 | 2,4-$Cl_2$-$C_6H_3$-O- | -$CH_2$- | E | | |
| 236 | 2-$CH_3$-$C_6H_4$-O- | -$CH_2$- | E | | |
| 237 | 4-$CH_3$-$C_6H_4$-O- | -$CH_2$- | E | | |
| 238 | 2-$OCH_3$-$C_6H_4$-O- | -$CH_2$- | E | | |
| 239 | 4-$OCH_3$-$C_6H_4$-O- | -$CH_2$- | E | | |
| 240 | 4-$CF_3$-$C_6H_4$-O- | -$CH_2$- | E | | |

| Nr. | $R^3$ | $(X)_n$ | Konfiguration | $^1H$-NMR | Fp. (°C) |
|---|---|---|---|---|---|
| 241 | $C_6H_5-O-$ | $-CH(CH_3)-$ | E | | |
| 242 | $C_6H_5-O-$ | $-CH_2-CH_2-$ | E | | |
| 243 | $2-Cl-C_6H_4-O-$ | $-CH_2-CH_2-$ | E | | |
| 244 | $4-Cl-C_6H_4-O-$ | $-CH_2-CH_2-$ | E | | |
| 245 | $2-CH_3-C_6H_4-O-$ | $-CH_2-CH_2-$ | E | | |
| 246 | $4-CH_3-C_6H_4-O-$ | $-CH_2-CH_2-$ | E | | |
| 247 | $2-OCH_3-C_6H_4-O-$ | $-CH_2-CH_2-$ | E | | |
| 248 | $4-OCH_3-C_6H_4-O-$ | $-CH_2-CH_2-$ | E | | |
| 249 | $4-t-C_4H_9-C_6H_4-O-$ | $-CH_2-CH_2-$ | E | | |
| 250 | $4-sec.-C_4H_9-C_6H_4-O-$ | $-CH_2-CH_2-$ | E | | |
| 251 | $C_6H_5-O-$ | $-(CH_2)_3-$ | E | | |
| 252 | $2-Cl-C_6H_4-O-$ | $-(CH_2)_3-$ | E | | |
| 253 | $4-Cl-C_6H_4-O-$ | $-(CH_2)_3-$ | E | | |
| 254 | $3-F-C_6H_4-O-$ | $-(CH_2)_3-$ | E | | |
| 255 | $4-F-C_6H_4-O-$ | $-(CH_2)_3-$ | E | | |
| 256 | $2-CH_3-C_6H_4-O-$ | $-(CH_2)_3-$ | E | | |
| 257 | $4-CH_3-C_6H_4-O-$ | $-(CH_2)_3-$ | E | | |
| 258 | $2-OCH_3-C_6H_4-O-$ | $-(CH_2)_3-$ | E | | |
| 259 | $4-OCH_3-C_6H_4-O-$ | $-(CH_2)_3-$ | E | | |
| 260 | $2,4-Cl_2-C_6H_3-O-$ | $-(CH_2)_3-$ | E | | |
| 261 | $4-Cl-C_6H_4-O-$ | $-CH(CH_3)-CH_2-CH_2-$ | E | | |
| 262 | $2-CF_3-C_6H_4-O-$ | $-(CH_2)_3-$ | E | | |
| 263 | $3-CF_3-C_6H_4-O-$ | $-(CH_2)_3-$ | E | | |
| 264 | $4-CF_3-C_6H_4-O-$ | $-(CH_2)_3-$ | E | | |
| 265 | $4-t-Butoxy-C_6H_4-O-$ | $-(CH_2)_3-$ | E | | |
| 266 | $2-CH_3-4-Cl-C_6H_3-O-$ | $-(CH_2)_3-$ | E | | |
| 267 | $4-C_2H_5-C_6H_4-O-$ | $-(CH_2)_3-$ | E | | |
| 268 | $4-iso-C_3H_7-C_6H_4-O-$ | $-(CH_2)_3-$ | E | | |
| 269 | $4-t-C_4H_9-C_6H_4-O-$ | $-(CH_2)_3-$ | E | | |

| Nr. | $R^3$ | $(X)_n$ | Konfiguration | $^1H$-NMR | Fp. (°C) |
|---|---|---|---|---|---|
| 270 | $C_6H_5-O-$ | $-CH_2-CH(CH_3)-CH_2-$ | E | | |
| 271 | $C_6H_5-O-$ | $-(CH_2)_4-$ | E | 1,88(m,4H); 2,45(t,2H); 3,74(s,3H); 3,88(s,3H); 4,01(t,2H); 5,09(s,2H); 7,18(m,9H); 7,64(s,1H). | Oel |
| 272 | $2-Cl-C_6H_4-O-$ | $-(CH_2)_4-$ | E | | |
| 273 | $4-Cl-C_6H_4-O-$ | $-(CH_2)_4-$ | E | | |
| 274 | $2,4-Cl_2-C_6H_3-O-$ | $-(CH_2)_4-$ | E | | |
| 275 | $2,6-Cl_2-C_6H_3-O-$ | $-(CH_2)_4-$ | E | | |
| 276 | $2-CH_3-C_6H_4-O-$ | $-(CH_2)_4-$ | E | | |
| 277 | $4-CH_3-C_6H_4-O-$ | $-(CH_2)_4-$ | E | | |
| 278 | $C_6H_5-O-$ | $-CH_2-CH_2-CH(CH_3)-CH_2-$ | E | | |
| 279 | $C_6H_5-O-$ | $-(CH_2)_5-$ | E | | |
| 280 | $3-Cl-C_6H_4-O-$ | $-(CH_2)_5-$ | E | | |
| 281 | $C_6H_5-O-$ | $-(CH_2)_3-CH(CH_3)-CH_2-$ | E | | |
| 282 | $C_6H_5-O-$ | $-(CH_2)_6-$ | E | | |
| 283 | $3-Cl-C_6H_5-O-$ | $-(CH_2)_6-$ | E | | |
| 284 | $C_6H_5-O-$ | $-(CH_2)_4-CH(CH_3)-CH_2-$ | E | | |
| 285 | A 1*) | - | E | 1.13, 1.18, 1.25, 1.30(4s,6H); 1.73 (s,6H); 1.88, 2.08(2m,1H); 3.70 (s,3H); 3.82(s,3H); 4.90, 5.45(2m,1H); 5,05(m,2H); 7,38(m,4H); 7.62(s,1H). | Oel |
| 286 | A 2*) | - | E | 1.88, 1.23, 1.27, 1.30(4s,6H); 1.13,1.88 (2d,1H); 2.03, 2.27(2m,1H); 3,70 (s,3H); 3.82(s,3H); 5.03(m,2H); 5.62, 6.30(2d,1H); 7.35(m,4H); 7.70(s,1H). | Oel |
| 287 | A 3*) | - | E | 1.19, 1.24, 1.28, 1.31(4s,6H); 1.68, 1.88 (2d,1H); 1.96, 2.19(2m,1H); 3.72 (s,3H); 3.83(s,3H); 5.04(m,2H); | Oel |

| Nr. | $R^3$ | $(X)_n$ | Konfiguration | $^1$H-NMR | Fp. (°C) |
|---|---|---|---|---|---|
| | | | | 6.16, 6.81(2d,1H); 7.32(m,4H); 7.60(s,1H). | |
| 288 | A 4*) | - | E | 1.24, 1.29, 1.32, 1.35(4s,6H); 1.79, 2.00(2d,1H); 2.17, 2.44,(2m,1H); 3,71(s,3H); 3,83(s,3H); 5.07 (m,2H); 6.16, 6.97(2d,1H); 7.36(m,4H); 7.60(s,1H). | Oel |
| 289 | A 5*) | - | E | 1.45, 1.49(2s,6H); 2.13(s,1H); 3.73(s,3H); 3.84(s,3H); 5.09(s,2H); 7,36(m,4H); 7,60(s,1H). | Oel |
| 290 | A 6*) | - | E | | |
| 291 | A 7*) | - | E | | |
| 292 | A 8*) | - | E | | |
| 293 | A 9*) | - | E | | |
| 294 | A 10*) | - | E | | |
| 295 | A 11*) | - | E | | |
| 296 | A 12*) | - | E | | |
| 297 | A 13*) | - | E | | |
| 298 | A 14*) | - | E | | |
| 299 | A 15*) | - | E | | |
| 300 | 2-Furyl | -CH=CH- | E | | Oel |
| 301 | N-Pyrrolyl | CH(iso-$C_3H_7$) | E | 0.77(d,3H); 0.98(d,3H); 2.42(sept.,1H); 3.69(s,3H); 3.80(s,3H); 4.18(d,1H); 5.03(s,2H); 6.20(m,2H); 6.83(m,2H); 7.31(m,4H); 7.58(s,1H). | Oel |
| 302 | 4-tert.-Butyl-$C_6H_4$ | $-CH_2-C(CH_3)=CH-CH=CH-$ | E | 1.33(s,9H); 2.22(s.3H); 3.43(s,2H); 3.72(s,3H); 3.87(s.3H); 5.13(s,2H); | Oel |

| Nr. | $R^3$ | $(X)_n$ | Konfiguration | $^1$H-NMR | Fp. (°C) |
|---|---|---|---|---|---|
| | | | | 5.88(d,1H); 6.10(m,1H); 6.45(d,1H); 7.35(m,8H); 7.63(s,1H). | |
| 303 | H | $-CH_2-CH(CH_3)-CH_2-CH(CH_3)-$ | E | | |
| 304 | H | $-CH_2-CH(CH_3)-CH_2-CH(CH_2H_5)-$ | E | 0.89(m,9H); 1.25(m,1H); 1.59(m,4H); 2.41(m,1H); 3.71(s,3H); 3.84(s,3H); 5.03(s,2H); 7.36(m,4H); 7.59(s,1H). | Oel |
| 305 | H | $-CH_2-CH(CH_3)-CH_2-CH(n-C_3H_7)-$ | E | | Oel |
| 306 | H | $-CH_2-CH(CH_3)-CH_2-CH_2-CH(i-C_3H_7)-$ | E | | Oel |
| 307 | H | $-CH_2-C(CH_3)_2-CH_2-CH(CH_3)-CH_2-$ | E | | Oel |
| 308 | H | $-(CH_2)_5-CH(C_2H_5)-$ | E | | |
| 309 | H | $-(CH_2)_5-CH(n-C_3-H_7)-$ | E | | |
| 354 | H | $-CH_2-O-CH_2-C(CH_3)_2-$ | E | | Oel |
| 355 | H | $-(CH_2)_4-O-CH_2-C(CH_3)_2-$ | E | | Oel |
| 356 | 1-Methylcyclohexyl | - | E | | Oel |
| 357 | $4-Cl-C_6H_4$ | $-CHCl-$ | E | | Oel |
| 358 | $4-Cl-C_6H_4$ | $-C(CH_3)_2-$ | E | | Oel |
| 359 | $C_6H_5$ | $-CH=CH-(CH_2)_4$ | E | | Oel |
| 360 | 1-Methylcyclopropyl | - | E | 0.65(m,2H); 1.22(m,2H); 1.30(s,3H); 3.70(s,3H); 3.83(s,3H); 5.00(s,2H); 7.14-7.48(m,4H); 7.60(s,1H). | Oel |
| 361 | 2-Methylcyclopropyl | - | E | | Oel |
| 362 | 2,2-Dichlorcyclopropyl | - | E | | Oel |
| 363 | 1-Methyl-2,2-dichlorcyclopropyl | - | E | | Oel |
| 364 | 2-Phenyl-cyclopropyl | - | E | | Oel |

| Nr. | $R^3$ | $(X)_n$ | Konfiguration | $^1H$-NMR | Fp. (°C) |
|---|---|---|---|---|---|
| 365 | 1-Phenylcyclopropyl | - | E | | 106-108 |
| 366 | 1-(2'-Chlorphenyl)-cyclopropyl | - | E | 1.21(m,2H); 1.76(m,2H); 3.68(s,3H); 3.77(s,3H); 5.00(s,2H); 7.08-7.40 (m,8H); 7.55(s,1H). | 100-101 |
| 367 | 1-(3'-Chlorphenyl)-cyclopropyl | - | E | | Oel |
| 368 | 1-(4'-Chlorphenyl)-cyclopropyl | - | E | | Oel |
| 369 | 1-(2',4'-Dichlorphenyl)-cyclopropyl | - | E | | |
| 370 | 1-(2',6'-Dichlorphenyl)-cyclopropyl | - | E | | |
| 371 | 1-(3',4'-Dichlorphenyl)-cyclopropyl | - | E | | |
| 372 | 1-(2'-Fluorphenyl)-cyclopropyl | - | E | | |
| 373 | 1-(3'-Fluorphenyl)-cyclopropyl | - | E | | |
| 374 | 1-(4'-Fluorphenyl)-cyclopropyl | - | E | | |
| 375 | 1-(4'-Bromphenyl)-cyclopropyl | - | E | | |
| 376 | 1-(2'-Methylphenyl)-cyclopropyl | - | E | | |
| 377 | 1-(3'-Methylphenyl)-cyclopropyl | - | E | | |
| 378 | 1-(4'-Methylphenyl)-cyclopropyl | - | E | | |
| 379 | 1-(3'4'-Dimethylphenyl)-cyclopropyl | - | E | | |
| 380 | 1-(4'-tert-Butylphenyl)-cyclopropyl | - | E | | |
| 381 | 1-(3'-Trifluormethylphenyl)-cyclopropyl | - | E | | |
| 382 | 1-(2'-Methoxyphenyl)-cyclopropyl | - | E | | |
| 383 | 1-(3'-Methoxyphenyl)-cyclopropyl | - | E | | |
| 384 | 1-(4'-Methoxyphenyl)-cyclopropyl | - | E | | 84-85 |
| 385 | 1-(2',4'-Dimethoxyphenyl)-cyclopropyl | - | E | | |
| 386 | 1-(2',6'-Dimethoxyphenyl)-cyclopropyl | - | E | | |
| 387 | 1-(3',4'-Dimethoxyphenyl)-cyclopropyl | - | E | | |
| 388 | 1-(3',4'-Dimethoxyphenyl)-cyclopropyl | - | E | | |

*) Formeln siehe vorhergehenden Text

Die angegebenen NMR-Daten geben die chemische Verschiebung (δ) der Protonen in ppm relativ zu Tetramethylsilan an. Als Lösungsmittel dient $CDCl_3$.

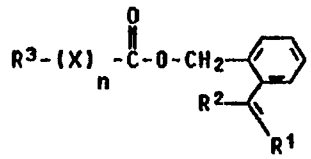

I

Tabelle 2: Verbindungen der Formel I

| Nr. | $R^1$ | $R^2$ | $R^3$ | $(X)_n$ | Konfiguration | Fp. (°C) |
|---|---|---|---|---|---|---|
| 310 | $OCH_3$ | CN | H | $-(CH_2)_4-CH(CH_3)-CH_2-$ | E | |
| 311 | $OCH_3$ | CN | $C_6H_5$ | $-(CH_2)_3-CH(CH_3)-CH_2-$ | E | |
| 312 | $OCH_3$ | CN | $A_2$*) | - | E/Z | Oel |
| 313 | $OCH_3$ | $CONH_2$ | H | $-(CH_2)_4-CH(CH_3)-CH_2-$ | E | |
| 314 | $OCH_3$ | $CONH_2$ | $C_6H_5$ | $-(CH_2)_3-CH(CH_3)-CH_2-$ | E | |
| 315 | $OCH_3$ | $CONH_2$ | $A_2$*) | - | E | |
| 316 | $SCH_3$ | $CO_2CH_3$ | H | $-(CH_2)_4-CH(CH_3)-CH_2-$ | E | |
| 317 | $SCH_3$ | $CO_2CH_3$ | $C_6H_5$ | $-(CH_2)_3-CH(CH_3)-Ch_2-$ | E | |
| 318 | $SCH_3$ | $CO_2CH_3$ | $A_2$*) | - | E | |
| 319 | $SCH_3$ | CN | H | $-(CH_2)_4-CH(CH_3)-CH_2-$ | E | |
| 320 | $SCH_3$ | CN | $C_6H_5$ | $-(CH_2)_3-CH(CH_3)-CH_2-$ | E | |
| 321 | $SCH_3$ | CN | $A_2$*) | - | E | |
| 322 | $SCH_3$ | $CONH_2$ | H | $-(CH_2)_4-CH(CH_3)-CH_2-$ | E | |
| 323 | $SCH_3$ | $CONH_2$ | $C_6H_5$ | $-(CH_2)_3-CH(CH_3)-CH_2-$ | E | |
| 324 | $SCH_3$ | $CONH_2$ | $A_2$*) | - | E | |
| 325 | Cl | $CO_2CH_3$ | H | $-(CH_2)_4-CH(CH_3)-CH_2-$ | E | |
| 326 | Cl | $CO_2CH_3$ | $C_6H_5$ | $-(CH_2)_3-CH(CH_3)-CH_2-$ | E | |
| 327 | Cl | $CO_2CH_3$ | $A_2$*) | - | E | |
| 328 | Cl | CN | H | $-(CH_2)_4-CH(CH_3)-CH_2-$ | E | |
| 329 | Cl | CN | $C_6H_5$ | $-(CH_2)_3-CH(CH_3)-CH_2-$ | E | |
| 330 | Cl | CN | $A_2$*) | - | E | |
| 331 | Cl | $CONH_2$ | H | $-(CH_2)_4-CH(CH_3)-CH_2-$ | E | |

| Nr. | $R^1$ | $R^2$ | $R^3$ | $(X)_n$ | Konfiguration | |
|---|---|---|---|---|---|---|
| 332 | Cl | $CONH_2$ | $C_6H_5$ | $-(CH_2)_3-CH(CH_3)-CH_2-$ | E | |
| 333 | Cl | $CONH_2$ | $A_2$*) | - | E | |
| 334 | $N(CH_3)_2$ | $CO_2CH_3$ | H | $-(CH_2)_4-CH(CH_3)-CH_2-$ | E | |
| 335 | $N(CH_3)_2$ | $CO_2CH_3$ | $C_6H_5$ | $-(CH_2)_3-CH(CH_3)-CH_2-$ | E | |
| 336 | $N(CH_3)_2$ | $CO_2CH_3$ | $A_2$*) | - | E | |
| 337 | $N(CH_3)_2$ | CN | H | $-(CH_2)_4-CH(CH_3)-CH_2-$ | E | |
| 338 | $N(CH_3)_2$ | CN | $C_6H_5$ | $-(CH_2)_3-CH(CH_3)-CH_2-$ | E | |
| 339 | $N(CH_3)_2$ | CN | $A_2$*) | - | E | |
| 340 | $N(CH_3)_2$ | $CONH_2$ | H | $-(CH_2)_4-CH(CH_3)-CH_2-$ | E | |
| 341 | $N(CH_3)_2$ | $CONH_2$ | $C_6H_5$ | $-(CH_2)_3-CH(CH_3)-CH_2-$ | E | |
| 342 | $N(CH_3)_2$ | $CONH_2$ | $A_2$*) | - | E | |
| 343 | $NHCH_3$ | $CO_2CH_3$ | H | $-CH_2)_4-CH(CH_3)-CH_2-$ | E | |
| 344 | $NHCH_3$ | $CO_2CH_3$ | $C_6H_5$ | $-CH_2)_4-CH(CH_3)-CH_2-$ | E | |
| 345 | $NHCH_3$ | $CO_2CH_3$ | $A_2$*) | - | E | |
| 346 | $NHCH_3$ | CN | H | $-CH_2)_4-CH(CH_3)-CH_2-$ | E | |
| 347 | $NHCH_3$ | CN | $C_6H_5$ | $-CH_2)_4-CH(CH_3)-CH_2-$ | E | |
| 348 | $NHCH_3$ | CN | $A_2$*) | - | E | |
| 349 | $NHCH_3$ | $CONH_2$ | H | $-CH_2)_4-CH(CH_3)-CH_2-$ | E | |
| 350 | $NHCH_3$ | $CONH_2$ | $C_6H_5$ | $-CH_2)_3-CH(CH_3)-CH_2-$ | E | |
| 351 | $NHCH_3$ | $CONH_2$ | $A_2$*) | - | E | |
| 352 | $OCH_3$ | CN | $2-F-C_6H_4$ | - | E/Z | 64-65 |
| 353 | $OCH_3$ | CN | 1-Methylcyclopropyl | - | E/Z | Oel |

*) Formel siehe vorhergehenden Text

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse – wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 34 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 226 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 286 werden in einer Mischung gelöst, die aus 40 Gew.-Teilen

Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 289 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 34 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 226 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 286 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 289 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 34 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester

heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,

N-(1,1,2,2-Tetrachlorethylthio)-tetrahydrophthalimid,
N-Trichlormethylthio-tetrahydrophthalimid,
N-Trichlormethylthio-phthalimid,

N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol, DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol, 2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoffe wurden N-Tridecyl-2,6-dimethylmorpholin (A) und α-(2-Benzoyloxyphenyl)-β-methoxyacrylsäureester (B) – bekannt aus DE 1 164 152 und aus EP 178 826 – benutzt.

Anwendungsbeispiel 1

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in einer Kammer mit hoher Luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80% Wirkstoff und 20% Emulgiermittel in der Trockensubstanz enthielten, tropfnaß gespritzt. Nach dem Abtrocknen des Spritzbelages wurden die Versuchspflanzen im Gewächshaus bei Temperaturen zwischen 20 und 22°C und 65 bis 70% relativer Luftfeuchte aufgestellt. Nach 8 Tagen wurde das Ausmaß der Rostpilzentwicklung auf den Blättern ermittelt.

Das Ergebnis zeigt, daß die Wirkstoffe 34, 192, 226, 286, 287, 289, 360, 361, 362, 363, 368 und 369 bei der Anwendung als 0,025%ige ( Gew.-%) Spritzbrühen eine bessere fungizide Wirkung zeigen (90%) als die bekannten Vergleichswirkstoffe A und B (50%).

Anwendungsbeispiel 2

Wirksamkeit gegen Pyrenophora teres

Gerstenkeimlinge der Sorte "Igri" wurden im Zweiblattstadium mit wäßrigen Suspensionen, die 80% Wirkstoff und 20% Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20–22°C und 70% relativer Luftfeuchtigkeit für weitere 5 Tage kultiviert. Dann wurde das Ausmaß der Symptomentwicklung ermittelt.

Das Ergebnis zeigt, daß die Wirkstoffe 1, 4, 11, 28, 34, 35, 71, 73, 83, 98, 101, 109, 154, 169, 192, 226, 271, 285, 286, 287, 288, 289, 304, 306, 355, 360, 361, 363 und 368 bei der Anwendung als 0,05%ige Spritzbrühe eine bessere fungizide Wirkung (90 %) zeigen als der bekannte Vergleichswirkstoff A (50%).

Anwendungsbeispiel 3

Wirksamkeit gegen Plasmopara viticola

Blätter von Topfreben der Sorte "Müller Thurgau" wurden mit wäßriger Spritzbrühe, die 80% Wirkstoff und 20% Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 4, 11, 23, 28, 32, 34, 35, 71, 73, 79, 81, 82, 83, 98, 101, 109, 154, 161, 169, 179, 192, 226, 271, 285, 286, 287, 288, 289, 304, 356, 357, 359, 360, 362, 363, 364, 368 und 369 bei der Anwendung als 0,05 %ige Spritzbrühe eine bessere fungizide Wirkung (90%) zeigen als der bekannte Vergleichswirkstoff A (50%).

**Patentansprüche**

1. Ortho-substituierte Carbonsäure-benzylester der allgemeinen Formel,

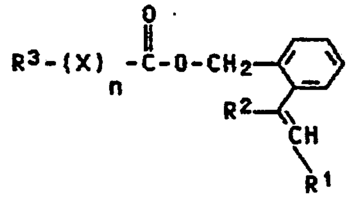 I

in der $R^1$ $C_1$-$C_4$-Alkoxy, $C_1$-$C_4$-Alkylthio, Halogen oder gegebenenfalls durch $C_1$-$C_4$-Alkyl einfach oder doppelt substituiertes Amino bedeutet,
$R^2$ $C_1$-$C_4$-Alkoxy-carbonyl, Cyano oder die Gruppe $CONH_2$ bedeutet,

$R^3$ Wasserstoff, Halogen, Cyano, Aryl, Aryloxy, wobei der aromatische Ring gegebenenfalls durch einen oder mehrere der folgenden Reste substituiert ist: $C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl, Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ einen gesättigten oder ungesättigten heterocyclischen Rest, $C_3$-$C_7$-Cycloalkyl, $C_5$-$C_6$-Cycloalkenyl, Adamantyl, Fluorenyl oder einen substituierten Cyclopropylrest bedeutet, der substituiert ist durch Methyl, Halogen, $C_1$-$C_2$-Halogenalkyl, $C_3$-$C_4$-Alkenyl, $C_2$-$C_4$-Halogenalkenyl, Methoxycarbonyl-$C_3C_4$-Alkenyl, Cyclopentylidenmethyl, Phenyl, Halogenphenyl, $C_1$-$C_2$-Alkoxyphenyl, $C_1$-$C_4$-Alkylphenyl,

X einen, gegebenenfalls durch Halogen oder Hydroxy substituierten, gegebenenfalls ungesättigten $C_1$-$C_{12}$-Alkylenrest bedeutet und

n die Zahlen 0 oder 1 bedeutet.

2. Verfahren zur Herstellung von ortho-substituierten Carbonsäure-benzyl-estern der allgemeinen Formel,

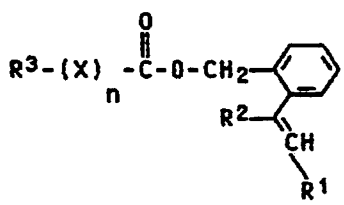

I

in der $R^1$ $C_1$-$C_4$-Alkoxy, $C_1$-$C_4$-Alkylthio, Halogen oder gegebenenfalls durch $C_1$-$C_4$-Alkyl einfach oder doppelt substituiertes Amino bedeutet,

$R^2$ $C_1$-$C_4$-Alkoxy-carbonyl, Cyano oder die Gruppe $CONH_2$ bedeutet,

$R^3$ Wasserstoff, Halogen, Cyano, Aryl, Aryloxy, wobei der aromatische Ring gegebenenfalls durch einen oder mehrere der folgenden Reste substituiert ist: $C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl, Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ einen gesättigten oder ungesättigten heterocyclischen Rest, $C_3$-$C_7$-Cycloalkyl, $C_5$-$C_6$-Cycloalkenyl, Adamantyl, Fluorenyl oder einen substituierten Cyclopropylrest bedeutet, der substituiert ist durch Methyl, Halogen, $C_1$-$C_2$-Halogenalkyl, $C_3$-$C_4$-Alkenyl, $C_2$-$C_4$-Halogenalkenyl, Methoxycarbonyl-$C_3C_4$-Alkenyl, Cyclopentylidenmethyl, Phenyl, Halogenphenyl, $C_1$-$C_2$-Alkoxyphenyl, $C_1$-$C_4$-Alkylphenyl,

X einen, gegebenenfalls durch Halogen oder Hydroxy substituierten, gegebenenfalls ungesättigten $C_1$-$C_{12}$-Alkylenrest bedeutet und

n die Zahlen 0 oder 1 bedeutet, dadurch gekennzeichnet, daß man ein Alkali-, Erdalkali- oder Ammoniumsalz einer Carbonsäure der Formel II mit einem ortho-substituierten Benzylbromid der Formel III, worin $R^1$, $R^2$, $R^3$, X und n die oben angegebene Bedeutung haben, in einem Lösungs- oder Verdünnungsmittel und gegebenenfalls in Gegenwart eines Katalysators umsetzt.

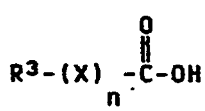

II

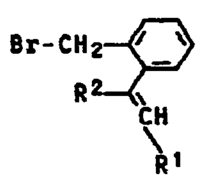

III

3. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge einer Verbindung der Formel

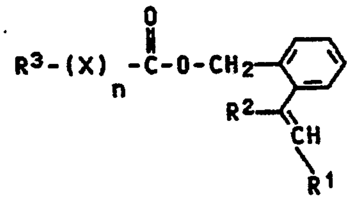

I

in der $R^1$ $C_1$-$C_4$-Alkoxy, $C_1$-$C_4$-Alkylthio, Halogen oder gegebenenfalls durch $C_1$-$C_4$-Alkyl einfach oder doppelt substituiertes Amino bedeutet,
$R^2$ $C_1$-$C_4$-Alkoxy-carbonyl, Cyano oder die Gruppe $CONH_2$ bedeutet,
$R^3$ Wasserstoff, Halogen, Cyano, Aryl, Aryloxy, wobei der aromatische Ring gegebenenfalls durch einen oder mehrere der folgenden Reste substituiert ist: $C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl, Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ einen gesättigten oder ungesättigten heterocyclischen Rest, $C_3$-$C_7$-Cycloalkyl, $C_5$-$C_6$-Cycloalkenyl, Adamantyl, Fluorenyl oder einen substituierten Cyclopropylrest bedeutet, der substituiert ist durch Methyl, Halogen, $C_1$-$C_2$-Halogenalkyl, $C_3$-$C_4$-Alkenyl, $C_2$-$C_4$-Halogenalkenyl, Methoxycarbonyl-$C_3$-$C_4$-Alkenyl, Cyclopentylidenmethyl, Phenyl, Halogenphenyl, $C_1$-$C_2$-Alkoxyphenyl, $C_1$-$C_4$-Alkylphenyl,
X einen, gegebenenfalls durch Halogen oder Hydroxy substituierten,
gegebenenfalls ungesättigten $C_1$-$C_{12}$-Alkylenrest bedeutet und
n die Zahlen 0 oder 1 bedeutet,
behandelt.

4. Fungizid, enthaltend einen inerten Trägerstoff und eine fungizid wirksame Menge einer Verbindung der Formel I

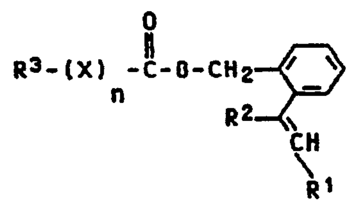

I

in der $R^1$ $C_1$-$C_4$-Alkoxy, $C_1$-$C_4$-Alkylthio, Halogen oder gegebenenfalls durch $C_1$-$C_4$-Alkyl einfach oder doppelt substituiertes Amino bedeutet,
$R^2$ $C_1$-$C_4$-Alkoxy-carbonyl, Cyano oder die Gruppe $CONH_2$ bedeutet,
$R^3$ Wasserstoff, Halogen, Cyano, Aryl, Aryloxy, wobei der aromatische Ring gegebenenfalls durch einen oder mehrere der folgenden Reste substituiert ist: $C_1$-$C_6$-Alkyl, $C_2$-$C_4$-Alkenyl, $C_1$-$C_2$-Halogenalkyl, $C_1$-$C_6$-Alkoxy, $C_1$-$C_4$-Alkoxy-$C_1$-$C_4$-alkyl, Aryl, Aryl-$C_1$-$C_2$-alkyl, Aryloxy, Aryloxy-$C_1$-$C_4$-alkyl, Aryloxy-$C_1$-$C_4$-alkoxy, Halogenaryloxy-$C_1$-$C_4$-alkoxy, Halogen, Halogen-$C_1$-$C_4$-alkoxy, $C_1$-$C_4$-Alkylthio, Thiocyanato, Cyano, Nitro, oder $R^3$ einen gesättigten oder ungesättigten heterocyclischen Rest, $C_3$-$C_7$-Cycloalkyl, $C_5$-$C_6$-Cycloalkenyl, Adamantyl, Fluorenyl oder einen substituierten Cyclopropylrest bedeutet, der substituiert ist durch Methyl, Halogen, $C_1$-$C_2$-Halogenalkyl, $C_3$-$C_4$-Alkenyl, $C_2$-$C_4$-Halogenalkenyl, Methoxycarbonyl-$C_3$-$C_4$-Alkenyl, Cyclopentylidenmethyl, Phenyl, Halogenphenyl, $C_1$-$C_2$-Alkoxyphenyl, $C_1$-$C_4$-Alkylphenyl,
X einen, gegebenenfalls durch Halogen oder Hydroxy substituierten, gegebenenfalls ungesättigten $C_1$-$C_{12}$-Alkylenrest bedeutet und
n die Zahlen 0 oder 1 bedeutet.

5. Verbindung der Formel I gemäß Anspruch 1, dadurch gekennzeichnet, daß $R^3$ Wasserstoff, $X_n$ 2-Methylhexylen und $R^1$ Methoxy und $R^2$ Methoxycarbonyl bedeuten.

6. Verbindung der Formel I gemäß Anspruch 1, dadurch gekennzeichnet, daß $R^3$ Phenyl, $X_n$ 2-Methylpentylen und $R^1$ Methoxy und $R^2$ Methoxycarbonyl bedeuten.

## Revendications

1. Carboxylates de benzyle substitués en position ortho, de la formule générale

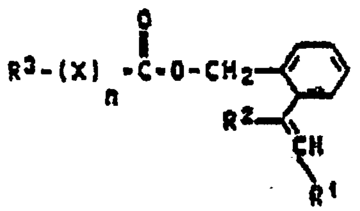

I

dans laquelle
$R^1$ représente une radical alcoxy en $C_1$–$C_4$, alkylthio en $C_1$–$C_4$, un atome d'halogène ou un radical amino éventuellement monosubstitué ou bisubstitué par des radicaux alkyle en $C_1$–$C_4$,
$R^2$ représente un radical alcoxy($C_1$–$C_4$)-carbonyle, cyano ou $CONH_2$,
$R^3$ représente un atome d'hydrogène, un atome d'halogène, un radical cyano, aryle, aryloxy, où le noyau aromatique peut éventuellement être substitué par un ou plusieurs des groupes suivants: alkyle en $C_1$–$C_6$, alcényle en $C_2$–$C_4$, halogénalkyle en $C_1$–$C_2$, alcoxy en $C_1$–$C_6$, alcoxy($C_1$–$C_4$)-alkyle en $C_1$–$C_4$, aryle, aryl-alkyle en $C_1$–$C_2$, aryloxy, aryloxy-alkyle en $C_1$–$C_4$, aryloxy-alcoxy en $C_1$–$C_4$, halogénaryloxy-alcoxy en $C_1$–$C_4$, alkylthio en $C_1$–$C_4$, thiocyanoto, cyano, nitro, ou bien $R^3$ représente un radical hétérocyclique saturé ou insaturé, cycloalkyle en $C_3$–$C_7$, cycloalcényle en $C_5$–$C_6$, adamantyle, fluorényle, ou cyclopropyle substitué qui est substitué par des radicaux méthyle, des atomes d'halogènes, des radicaux halogénalkyle en $C_1$–$C_2$, alcényle en $C_3$–$C_4$, halogéalcényle en $C_2$–$C_4$, méthoxycarbonyl-alcényle ($C_3$–$C_4$), cyclopentylidèneméthyle, phényle, halogénophényle, alcoxy($C_1$–$C_2$)-phényle, alkyle ($C_1$–$C_4$)phényle,
X représente un radical un radical alkylènael en $C_1$–$C_{12}$, éventuellement insaturé et éventuellement substitué par des atomes d'halogènes ou des radicaux hydroxyle et
n est égal é 0 ou é 1.

2. Procédé de préparation de carboxylate de benzyle substitué en position ortho, de la formule générale

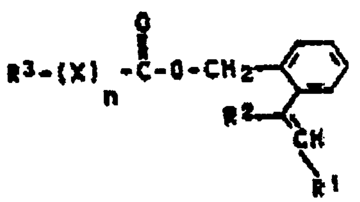

I

dans laquelle
$R^1$ représente une radical alcoxy en $C_1$–$C_4$, alkylthio en $C_1$–$C_4$, un atome d'halogène ou un radical amino éventuellement monosubstitué ou bisubstitué par des radicaux alkyle en $C_1$–$C_4$,
$R^2$ représente un radical alcoxy($C_1$–$C_4$)-carbonyle, cyano ou $CONH_2$,
$R^3$ représente un atome d'hydrogène, un atome d'halogène, un radical cyano, aryle, aryloxy, où le noyau aromatique peut éventuellement être substitué par un ou plusieurs des groupes suivants: alkyle en $C_1$–$C_6$, alcényle en $C_2$–$C_4$, halogénalkyle en $C_1$–$C_2$, alcoxy en $C_1$–$C_6$, alcoxy($C_1$–$C_4$)-alkyle en $C_1$–$C_4$, aryle, aryl-alkyle en $C_1$–$C_2$, aryloxy, aryloxy-alkyle en $C_1$–$C_4$, aryloxy-alcoxy en $C_1$–$C_4$, halogénaryloxy-alcoxy en $C_1$–$C_4$, atomes d'halogènes, halogéno-alcoxy en $C_1$–$C_4$, alkylthio en $C_1$–$C_4$, thiocyanoto, cyano, nitro, ou bien $R^3$ représente un radical hétérocyclique saturé ou insaturé, cycloalkyle en $C_3$–$C_7$, cycloalcényle en $C_5$–$C_6$, adamantyle, fluorényle, ou cyclopropyle substitué qui est substitué par des radicaux méthyle, des atomes d'halogènes, des radicaux halogénalkyle en $C_1$–$C_2$, alcényle en $C_3$–$C_4$, halogéalcényle en $C_2$–$C_4$, méthoxycarbonyl-alcényle ($C_3$–$C_4$), cyclopentylidèneméthyle, phényle, halogénophényle, alcoxy($C_1$–$C_2$)-phényle, alkyle ($C_1$–$C_4$)phényle,
X représente un radical un radical alkylène en $C_1$–$C_{12}$, éventuellement insaturé et éventuellement substitué par des atomes d'halogènes ou des radicaux hydroxyle et
n est égal à 0 ou à 1.
caractérisé en ce que l'on fait réagir un sel de métal alcalin, de métal alcalino-terreux ou d'ammonium d'un acide carboxylique de la formule II avec un bromure de benzyle substitué en position ortho de la formule III dans laquelle, $R^1$, $R^2$, $R^3$, X et n possèdent les significations qui leur ont été attribuées dans la revendication 1, dans un solvant ou un diluant et éventuellement en présence d'un catalyseur:

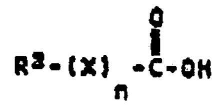

II

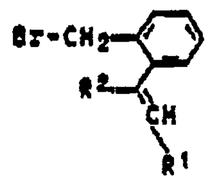

III

3. Procédé pour lutter contre des champignons, caractérisé en ce que l'on traite la terre, les semences, les plantes ou les matériaux menacés d'une attaque par des champignons par une proportion efficace du point de vue fongicide d'un composé de la formule

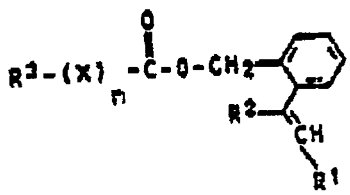

I

dans laquelle
$R^1$ représente une radical alcoxy en $C_1$–$C_4$, alkylthio en $C_1$–$C_4$, un atome d'halogène ou un radical amino éventuellement monosubstitué ou bisubstitué par des radicaux alkyle en $C_1$–$C_4$,
$R^2$ représente un radical alcoxy($C_1$–$C_4$)-carbonyle, cyano ou $CONH_2$,
$R^3$ représente un atome d'hydrogène, un atome d'halogène, un radical cyano, aryle, aryloxy, où le noyau aromatique peut éventuellement être substitué par un ou plusieurs des groupes suivants: alkyle en $C_1$–$C_6$, alcényle en $C_2$–$C_4$, halogénalkyle en $C_1$–$C_2$, alcoxy en $C_1$–$C_6$, alcoxy($C_1$–$C_4$)-alkyle en $C_1$–$C_4$, aryle, aryl-alkyle en $C_1$–$C_2$, aryloxy, aryloxy-alkyle en $C_1$–$C_4$, aryloxy-alcoxy en $C_1$–$C_4$, halogénaryloxy-alcoxy en $C_1$–$C_4$, atomes d'halogènes, halogéno-alcoxy en $C_1$–$C_4$, alkylthio en $C_1$–$C_4$, thiocyanoto, cyano, nitro, ou bien $R^3$ représente un radical hétérocyclique saturé ou insaturé, cycloalkyle en $C_3$–$C_7$, cycloalcényle en $C_5$–$C_6$, adamantyle, fluorényle, ou cyclopropyle substitué qui est substitué par des radicaux méthyle, des atomes d'halogènes, des radicaux halogénalkyle en $C_1$–$C_2$, alcényle en $C_3$–$C_4$, halogénalcényle en $C_2$–$C_4$, méthoxycarbonyl-alcényle ($C_3$–$C_4$), cyclopentylidèneméthyle, phényle, halogénophényle, alcoxy($C_1$–$C_2$)-phényle, alkyle ($C_1$–$C_4$)phényle,
X représente un radical représente un radical alkylène en $C_1$–$C_{12}$,
éventuellement insaturé et éventuellement substitué par des atomes d'halogènes ou des radicaux hydroxyle et
n est égal à 0 ou à 1.

4. Fongicide, contenant un véhicule ou support inerte et

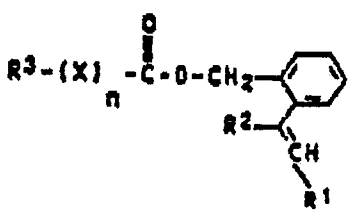

I

dans laquelle
$R^1$ représente une radical alcoxy en $C_1$–$C_4$, alkylthio en $C_1$–$C_4$, un atome d'halogène ou un radical amino éventuellement monosubstitué ou bisubstitué par des radicaux alkyle en $C_1$–$C_4$,
$R^2$ représente un radical alcoxy($C_1$–$C_4$)-carbonyle, cyano ou $CONH_2$,
$R^3$ représente un atome d'hydrogène, un atome d'halogène, un radical cyano, aryle, aryloxy, où le noyau aromatique peut éventuellement être substitué par un ou plusieurs des groupes suivants: alkyle en $C_1$–$C_6$, alcényle en $C_2$–$C_4$, halogénalkyle en $C_1$–$C_2$, alcoxy en $C_1$–$C_6$, alcoxy($C_1$–$C_4$)-alkyle en $C_1$–$C_4$, aryle, aryl-alkyle en $C_1$–$C_2$, aryloxy, aryloxy-alkyle en $C_1$–$C_4$, aryloxy-alcoxy en $C_1$–$C_4$, halogén-

aryloxy-alcoxy en $C_1$–$C_4$, atomes d'halogènes, halogéno-alcoxy en $C_1$–$C_4$, alkylthio en $C_1$–$C_4$, thiocyanoto, cyano, nitro, ou bien $R^3$ représente un radical hétérocyclique saturé ou insaturé, cycloalkyle en $C_3$–$C_7$, cycloalcényle en $C_5$–$C_6$, adamantyle, fluorényle, ou cyclopropyle substitué qui est substitué par des radicaux méthyle, des atomes d'halogènes, des radicaux halogénalkyle en $C_1$–$C_2$, alcényle en $C_3$–$C_4$, halogénalcényle en $C_2$–$C_4$, méthoxycarbonyl-alcényle ($C_3$–$C_4$), cyclopentylidèneméthyle, phényle, halogénophényle, alcoxy($C_1$–$C_2$)-phényle, alkyle ($C_1$–$C_4$)phényle,
X représente un radical représente un radical alkylène en $C_1$–$C_{12}$, éventuellement insaturé et éventuellement substitué par des atomes d'halogènes ou des radicaux hydroxyle et
n est égal à 0 ou à 1.

5. Composé de la formule I selon la revendication 1, caractérisé en ce que $R^3$ représente un atome d'hydrogène, $X_n$ représente le radical 2-méthylhexyléne et $R_1$ et $R_2$ représente des radicaux méthoxy.

6. Composé de la formule I suivant la revendication 1, caractérisé en ce que $R^3$ représente le radical phényle, $X_n$ représente le radical 2-méthylpentylène et $R_1$ et $R_2$ représente des radicaux méthoxy.

## Claims

1. An ortho-substituted benzyl carboxylate of the general formula

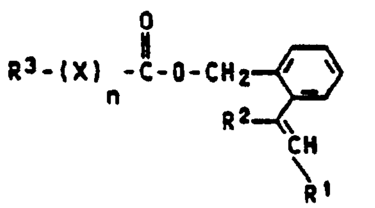

I

where $R^1$ is $C_1$–$C_4$-alkoxy, $C_1$–$C_4$-alkylthio or halogen or is amino which may be monosubstituted or disubstituted by $C_1$–$C_4$-alkyl, $R^2$ is $C_1$–$C_4$-alkoxycarbonyl, cyano or $CONH_2$, $R^3$ is hydrogen, halogen, cyano, aryl or aryloxy, in which the aromatic ring is unsubstituted or substituted by one or more of the following radicals: $C_1$–$C_6$-alkyl, $C_2$–$C_4$-alkenyl, $C_1$–$C_2$-haloalkyl, $C_1$–$C_6$-alkoxy, $C_1$–$C_4$-alkoxy-$C_1$–$C_4$-alkyl, aryl, aryl-$C_1$–$C_2$-alkyl, aryloxy, aryloxy-$C_1$–$C_4$-alkyl, aryloxy-$C_1$–$C_4$-alkoxy, haloaryloxy-$C_1$–$C_4$-alkoxy, halogen, halo-$C_1$–$C_4$-alkoxy, $C_1$–$C_4$-alkylthio, thiocyanato, cyano or nitro, or $R^3$ is a saturated or unsaturated heterocyclic radical, $C_3$–$C_7$-cycloalkyl, $C_5$–$C_6$-cycloalkenyl, adamantyl, fluorenyl, or a cyclopropyl radical which is substituted by methyl, halogen, $C_1$–$C_2$-haloalkyl, $C_3$–$C_4$-alkenyl, $C_2$–$C_4$-haloalkenyl, methoxycarbonyl-$C_3$–$C_4$-alkenyl, cyclopentylidenemethyl, phenyl, halophenyl, $C_1$–$C_2$-alkoxyphenyl or $C_1$–$C_4$-alkylphenyl, X is unsaturated or saturated $C_1$–$C_{12}$-alkylene which is unsubstituted or substituted by halogen or hydroxyl, and n is 0 or 1.

2. A process for preparing an ortho-substituted benzyl carboxylate of the general formula

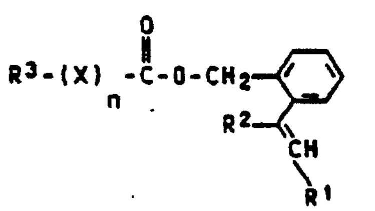

I

where $R^1$ is $C_1$–$C_4$-alkoxy, $C_1$–$C_4$-alkylthio or halogen or is amino which may be monosubstituted or disubstituted by $C_1$–$C_4$-alkyl, $R^2$ is $C_1$–$C_4$-alkoxycarbonyl, cyano or $CONH_2$, $R^3$ is hydrogen, halogen, cyano, aryl or aryloxy, in which the aromatic ring is unsubstituted or substituted by one or more of the following radicals: $C_1$–$C_6$-alkyl, $C_2$–$C_4$-alkenyl, $C_1$–$C_2$-haloalkyl, $C_1$–$C_6$-alkoxy, $C_1$–$C_4$-alkoxy-$C_1$–$C_4$-alkyl, aryl, aryl-$C_1$–$C_2$-alkyl, aryloxy, aryloxy-$C_1$–$C_4$-alkyl, aryloxy-$C_1$–$C_4$-alkoxy, haloaryloxy-$C_1$–$C_4$-alkoxy, halogen, halo-$C_1$–$C_4$-alkoxy, $C_1$–$C_4$-alkylthio, thiocyanato, cyano or nitro, or $R^3$ is a saturated or unsaturated heterocyclic radical, $C_3$–$C_7$-cycloalkyl, $C_5$–$C_6$-cycloalkenyl, adamantyl, fluorenyl, or a cyclopropyl radical which is substituted by methyl, halogen, $C_1$–$C_2$-haloalkyl, $C_3$–$C_4$-alkenyl, $C_2$–$C_4$-haloalkenyl, methoxycarbonyl-$C_3$–$C_4$-alkenyl, cyclopentylidenemethyl, phenyl, halophenyl, $C_1$–$C_2$-alkoxyphenyl or $C_1$–$C_4$-alkylphenyl, X is unsaturated or saturated $C_1$–$C_{12}$-alkylene which is unsubstituted or substituted by halogen or hydroxyl, and n is 0 or 1, which comprises reacting an alkali metal salt, alkaline earth metal salt or ammonium salt of a carboxylic acid of the formula II with an ortho-substituted benzyl bromide of the formula III, where $R^1$, $R^2$, $R^3$, X and n have the abovementioned meanings, in a solvent or diluent and in the presence or absence of a catalyst.

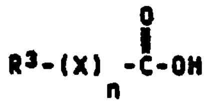

II

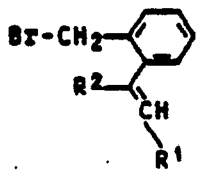

III

3. A method for controlling fungi, wherein the fungi or the materials, plants, seed or the soil threatened by fungal attack are treated with a fungicidally effective amount of a compound of the formula

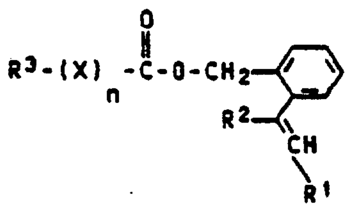

I

where $R^1$ is $C_1$–$C_4$-alkoxy, $C_1$–$C_4$-alkylthio or halogen or is amino which may be monosubstituted or disubstituted by $C_1$–$C_4$-alkyl, $R^2$ is $C_1$–$C_4$-alkoxycarbonyl, cyano or $CONH_2$, $R^3$ is hydrogen, halogen, cyano, aryl or aryloxy, in which the aromatic ring is unsubstituted or substituted by one or more of the following radicals: $C_1$–$C_6$-alkyl, $C_2$–$C_4$-alkenyl, $C_1$–$C_2$-haloalkyl, $C_1$–$C_6$-alkoxy, $C_1$–$C_4$-alkoxy-$C_1$–$C_4$-alkyl, aryl, aryl-$C_1$–$C_2$-alkyl, aryloxy, aryloxy-$C_1$–$C_4$-alkyl, aryloxy-$C_1$–$C_4$-alkoxy, haloaryloxy-$C_1$–$C_4$-alkoxy, halogen, halo-$C_1$–$C_4$-alkoxy, $C_1$–$C_4$-alkylthio, thiocyanato, cyano or nitro, or $R^3$ is a saturated or unsaturated heterocyclic radical, $C_3$–$C_7$-cycloalkyl, $C_5$–$C_6$-cycloalkenyl, adamantyl, fluorenyl, or a cyclopropyl radical which is substituted by methyl, halogen, $C_1$–$C_2$-haloalkyl, $C_3$–$C_4$-alkenyl, $C_2$–$C_4$-haloalkenyl, methoxycarbonyl-$C_3$–$C_4$-alkenyl, cyclopentylidenemethyl, phenyl, halophenyl, $C_1$–$C_2$-alkoxyphenyl or $C_1$–$C_4$-alkylphenyl, X is unsaturated or saturated $C_1$–$C_{12}$-alkylene which is unsubstituted or substituted by halogen or hydroxyl, and n is 0 or 1.

4. A fungicide containing an inert carrier and a fungicidally effective amount of a compound of the formula

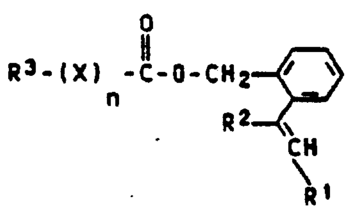

I

where $R^1$ is $C_1$–$C_4$-alkoxy, $C_1$–$C_4$-alkylthio or halogen or is amino which may be monosubstituted or disubstituted by $C_1$–$C_4$-alkyl, $R^2$ is $C_1$–$C_4$-alkoxycarbonyl, cyano or $CONH_2$, $R^3$ is hydrogen, halogen, cyano, aryl or aryloxy, in which the aromatic ring is unsubstituted or substituted by one or more of the following radicals: $C_1$–$C_6$-alkyl, $C_2$–$C_4$-alkenyl, $C_1$–$C_2$-haloalkyl, $C_1$–$C_6$-alkoxy, $C_1$–$C_4$-alkoxy-$C_1$–$C_4$-alkyl, aryl, aryl-$C_1$–$C_2$-alkyl, aryloxy, aryloxy-$C_1$–$C_4$-alkyl, aryloxy-$C_1$–$C_4$-alkoxy, haloaryloxy-$C_1$–$C_4$-alkoxy, halogen, halo-$C_1$–$C_4$-alkoxy, $C_1$–$C_4$-alkylthio, thiocyanato, cyano or nitro, or $R^3$ is a saturated or unsaturated heterocyclic radical, $C_3$–$C_7$-cycloalkyl, $C_5$–$C_6$-cycloalkenyl, adamantyl, fluorenyl, or a cyclopropyl radical which is substituted by methyl, halogen, $C_1$–$C_2$-haloalkyl, $C_3$–$C_4$-alkenyl, $C_2$–$C_4$-haloalkenyl, methoxycarbonyl-$C_3$–$C_4$-alkenyl, cyclopentylidenemethyl, phenyl, halophenyl, $C_1$–$C_2$-alkoxyphenyl or $C_1$–$C_4$-alkylphenyl, X is unsaturated or saturated $C_1$–$C_{12}$-alkylene which is unsubstituted or substituted by halogen or hydroxyl, and n is 0 or 1.

5. A compound of the formula I as claimed in claim 1, wherein $R^3$ is hydrogen, $X_n$ is 2-methylhexylene and $R^1$ and $R^2$ are methoxy.

6. A compound of the formula I as claimed in claim 1, wherein $R^3$ is phenyl, $X_n$ is 2-methylpentylene and $R^1$ and $R^2$ are methoxy.